(19) Europäisches Patentamt
European Patent Office
Office européen des brevets

(11) EP 3 413 979 B1

(12) **FASCICULE DE BREVET EUROPEEN**

(45) Date de publication et mention de la délivrance du brevet:
**29.07.2020 Bulletin 2020/31**

(21) Numéro de dépôt: **17704755.2**

(22) Date de dépôt: **10.02.2017**

(51) Int Cl.:
*A61Q 5/00* (2006.01) *A61Q 17/04* (2006.01)
*A61Q 19/00* (2006.01) *A61K 8/81* (2006.01)
*A61Q 19/08* (2006.01) *A61K 8/92* (2006.01)
*A61K 8/06* (2006.01) *A61K 8/11* (2006.01)

(86) Numéro de dépôt international:
**PCT/EP2017/053063**

(87) Numéro de publication internationale:
**WO 2017/137597 (17.08.2017 Gazette 2017/33)**

(54) **EMULSIONS HUILE-DANS-EAU DONT LA PHASE GRASSE EST SOUS FORME D'UN MÉLANGE DE GOUTTES DE DIFFÉRENTES TAILLES**

W/O EMULSION MIT EINER FETT-PHASE, DIE DURCH EINE MISCHUNG VON TROPFEN UNTERSCHIEDLICHEN DURCHMESSERS GEFORMT WIRD

W/O EMULSION HAVING A FATTY PHASE WHICH IS FORMED BY A MIXTURE OF DOPS HAVING A DIFFERENT DIAMETER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.02.2016 FR 1651172**

(43) Date de publication de la demande:
**19.12.2018 Bulletin 2018/51**

(73) Titulaire: **Capsum**
**13013 Marseille (FR)**

(72) Inventeurs:
- **GOUTAYER, Mathieu**
  **35400 Saint Malo (FR)**
- **PAFUMI, Yan Eric**
  **13120 Gardanne (FR)**
- **CHATRY, Lucie**
  **05380 Chateauroux-les-Alpes (FR)**
- **BALBUSQUIER, Hélène**
  **12850 Sainte- Radegonde (FR)**

(74) Mandataire: **Lavoix**
**2, place d'Estienne d'Orves**
**75441 Paris Cedex 09 (FR)**

(56) Documents cités:
**WO-A1-2015/055748 WO-A2-2012/120043**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 413 979 B1

## Description

La présente invention a pour objet des émulsions huile-dans-eau dont la phase grasse est sous forme d'un mélange de gouttes de différentes tailles, ainsi que leurs procédés de préparation.

La présente invention a également pour objet l'utilisation cosmétique des émulsions susmentionnées.

Il existe à ce jour des dispersions de gouttes d'une phase grasse dispersée dans une phase aqueuse, notamment décrites dans les demandes WO2012/120043, FR2972367 et FR2976824. Ces dispersions sont notamment obtenues à l'aide d'un procédé microfluidique.

Pour améliorer la résistance mécanique de telles dispersions, la Demanderesse a mis au point des dispersions stables de gouttes dans laquelle un agent gélifiant a été ajouté dans la phase grasse dispersée.

Il est toutefois observé des difficultés pour formuler de telles gouttes, en particulier gélifiées, dans certains types de compositions cosmétiques.

En effet, les compositions cosmétiques sous forme d'émulsion nécessitent la mise en œuvre de quantités plus ou moins élevées de tensioactifs pour assurer une stabilité dans le temps satisfaisante desdites émulsions.

Or, comme montré plus loin, il a été observé que la présence de tensioactif(s) dans une crème cosmétique sous forme d'émulsion comprenant les gouttes susmentionnées, en particulier gélifiées, entraîne une instabilité desdites gouttes.

Plus particulièrement, on constate un mûrissement compositionnel important se traduisant par une migration d'une partie de la phase huileuse des gouttes susmentionnées vers les micelles de tensioactifs localisés dans la crème. Ceci a pour effet une diminution en taille des gouttes et, pour les gouttes gélifiées, une augmentation de leur dureté (augmentation de la concentration locale en gélifiant huileux), ce qui dégrade fortement les propriétés organoleptiques de la composition.

Il existe donc un besoin de disposer de compositions cosmétiques, notamment de crèmes « riches » (notamment dont la teneur en phase grasse est élevée, à savoir supérieure à 10% en poids par rapport au poids total de la composition), qui, en présence de gouttes, en particulier de gouttes gélifiées, n'entrainent pas une instabilité desdites gouttes incorporées.

La présente invention a donc pour but de fournir une composition, notamment cosmétique, comprenant des gouttes, en particulier des gouttes gélifiées, permettant d'éviter le phénomène de mûrissement compositionnel susmentionné.

La présente invention a également pour but de fournir une composition comprenant des gouttes, en particulier des gouttes gélifiées, dont les propriétés organoleptiques sont maintenues, même dans des crèmes riches, et présentant une attractivité visuelle.

La présente invention a également pour but de fournir une composition comprenant des gouttes qui permettent une micro-diffusion prolongée pour une action continue au cœur de la peau d'actifs, en particulier d'agents hydratants et/ou d'agents anti-âge.

La présente invention a également pour but de fournir une composition comprenant des gouttes, en particulier des gouttes gélifiées, permettant de maintenir la résistance mécanique desdites gouttes.

Qui plus est, la mise à disposition de compositions cosmétiques présentant un visuel et un ressenti à l'application inédits demeure un objectif constant.

Ainsi, la présente invention concerne une composition, notamment cosmétique, sous la forme d'une émulsion huile-dans-eau, comprenant une phase aqueuse continue et une phase grasse dispersée sous forme de gouttes (G1) et (G2),

lesdites gouttes (G1) comprenant une phase grasse et une écorce formée d'au moins un polymère anionique (PA1) et d'au moins un polymère cationique (PC1), la taille des gouttes (G1) étant inférieure à 500 $\mu$m, et

lesdites gouttes (G2) comprenant une phase grasse et une écorce, ladite écorce étant formée d'au moins un polymère anionique (PA2), identique ou différent de (PA1), et d'au moins un polymère cationique (PC2), identique ou différent de (PC1), la taille des gouttes (G2) étant supérieure à 500 $\mu$m.

De préférence, les gouttes (G2) comprennent en outre au moins un agent gélifiant.

Les compositions selon l'invention comprennent donc une phase grasse sous forme d'un mélange de gouttes dont les gammes de tailles sont différentes. Comme expliqué après, la phase grasse dispersée correspond à un mélange de petites gouttes (G1), le cas échéant gélifiées, et de grandes gouttes (G2), de préférence gélifiées.

Les compositions selon l'invention peuvent également être décrites comme comprenant des gouttes (G2), ou grandes gouttes, dans une phase continue comprenant des gouttes (G1), ou petites gouttes, telles que définies ci-dessus. Ainsi, les compositions selon l'invention peuvent être caractérisées comme une émulsion huile-dans-eau comprenant au moins deux phases grasses, la première phase grasse étant figurée par les gouttes (G1) et la deuxième phase grasse étant figurée par les gouttes (G2), de préférence gélifiées.

Les compositions selon l'invention sont donc des mélanges de populations de gouttes de tailles différentes ce qui constitue une originalité galénique, notamment dans le domaine cosmétique.

Une composition selon l'invention est particulièrement avantageuse sur le plan visuel et sensoriel.

Sur le plan visuel, le consommateur est face à une composition comprenant des gouttes (G2) visibles à l'œil nu. L'intérêt de ce visuel attractif est encore exacerbé lorsqu'au moins les gouttes (G2) sont colorées.

Sur le plan sensoriel, la texture des compositions selon l'invention se différencie des émulsions « classiques » stabilisées par des tensioactifs. En effet, la présence des gouttes (G1) confère une texture unique, légère et volubile, procurant une application en deux temps. Plus particulièrement, l'émulsion reposant sur les gouttes (G1) s'étale facilement sur la peau. Les premiers instants d'application sont très aqueux avec un effet cassant marqué. Puis, le ressenti évolue vers un voile huileux qui s'estompe pour laisser une peau légère et hydratée. Cette texture est particulièrement avantageuse et surprenante pour l'homme du métier au vu de l'absence de tensioactifs dans ces émulsions.

Egalement, l'application sur une matière kératinique, en particulier la peau, d'une composition selon l'invention conduit au moment de son étalement à un cisaillement des gouttes (G2). L'effet sensoriel procuré par cette application se traduit par le fait de sentir littéralement les gouttes (G2) fondre sous l'effet de l'étalement et un effet huileux exacerbé. Cet effet sensoriel est encore exacerbé lorsque les gouttes (G2) sont gélifiées.

Dans le cadre de la présente invention, les émulsions susmentionnées peuvent être désignées indifféremment par le terme "dispersions".

Une goutte selon l'invention est composée d'un cœur, aussi appelé intérieur de la goutte, entouré d'une écorce, qui isole l'intérieur de la goutte de la phase continue de l'émulsion.

Avantageusement, le pH d'une composition selon l'invention est typiquement compris de 5,0 à 7,0.

***Taille des gouttes (G1) et (G2)***

Dans le cadre de la présente invention, le terme "taille" désigne le diamètre, notamment le diamètre moyen, des gouttes (G1) et (G2).

La phase grasse des compositions selon l'invention est donc constituée d'une combinaison de gouttes (G1) et (G2) de différentes tailles. On pourra désigner les gouttes (G1) comme des gouttes de petite taille (ou petites gouttes) et les gouttes (G2) comme des gouttes de grande taille (ou grandes gouttes).

Selon l'invention, la taille des gouttes (G1) est inférieure à 500 μm, de préférence inférieure à 400 μm, en particulier inférieure à 300 μm, mieux inférieure à 200 μm, en particulier inférieure à 100 μm, voire inférieure à 20 μm, et mieux inférieure à 10 μm. Préférentiellement, la taille des gouttes (G1) est comprise entre 0,1 et 200 μm, de préférence entre 0,25 et 100 μm, en particulier entre 0,5 μm et 50 μm, de préférence entre 1 μm et 20 μm, et mieux entre 1 μm et 10 μm, voire entre 3 μm et 5 μm.

Ces gouttes de taille réduite permettent d'avoir un effet sur la texture. En effet, une composition, formée de gouttes finement dispersées, présente des qualités d'onctuosité améliorées.

Selon l'invention, la taille des gouttes (G2) est supérieure à 500 μm, de préférence supérieure à 600 μm, en particulier supérieure à 700 μm, et mieux supérieure à 800 μm. Préférentiellement, la taille des gouttes (G2) est comprise entre 500 μm et 3 000 μm, de préférence entre 510 μm et 2500 μm, mieux entre 600 μm et 2000 μm, et plus particulièrement entre 700 μm et 1200 μm.

De préférence, les gouttes (G2), voire les gouttes (G1), présentent une distribution de taille uniforme. De préférence, les gouttes (G2) forment une population de gouttes monodispersées, notamment telles qu'elles possèdent un diamètre moyen $\overline{D}$ compris de 500 μm à 3 000 μm et un coefficient de variation Cv inférieur à 10%, voire inférieur à 3%.

Dans le cadre de la présente description, on entend par "gouttes monodispersées" le fait que la population de gouttes de la phase dispersée selon l'invention possède une distribution de taille uniforme. Des gouttes monodispersées présentent une bonne monodispersité. A l'inverse, des gouttes présentant une mauvaise monodispersité sont dites "polydispersées".

Selon un mode, le diamètre moyen $\overline{D}$ des gouttes est par exemple mesuré par analyse d'une photographie d'un lot constitué de N gouttes, par un logiciel de traitement d'image (Image J). Typiquement, selon cette méthode, le diamètre est mesuré en pixel, puis rapporté en μm, en fonction de la dimension du récipient contenant les gouttes de la dispersion.

De préférence, la valeur de N est choisie supérieure ou égale à 30, de sorte que cette analyse reflète de manière statistiquement significative la distribution de diamètres des gouttes de ladite émulsion.

On mesure le diamètre Di de chaque goutte, puis on obtient le diamètre moyen $\overline{D}$ en calculant la moyenne arithmétique de ces valeurs :

$$\overline{D} = \frac{1}{N}\sum_{i=1}^{N} D_i$$

A partir de ces valeurs $D_i$, on peut également obtenir l'écart-type σ des diamètres des gouttes de la dispersion :

$$\sigma = \sqrt{\frac{\sum_{i=1}^{N}\left(D_i - \overline{D}\right)^2}{N}}$$

L'écart-type σ d'une dispersion reflète la répartition des diamètres $D_i$ des gouttes de la dispersion autour du diamètre moyen $\overline{D}$.

En connaissant le diamètre moyen $\overline{D}$ et l'écart-type σ d'une dispersion, on peut déterminer que l'on trouve 95,4% de la population de gouttes dans l'intervalle de diamètres $[\overline{D}-2\sigma;\overline{D}+2\sigma]$ et que l'on trouve 68,2% de la population dans l'intervalle $[\overline{D}-\sigma;\overline{D}+\sigma]$.

Pour caractériser la monodispersité de la dispersion selon ce mode de l'invention, on peut calculer le coefficient de variation :

$$C_v = \frac{\sigma}{\overline{D}}$$

Ce paramètre reflète la répartition des diamètres des gouttes en fonction du diamètre moyen de celles-ci.

Le coefficient de variation Cv des diamètres des gouttes selon ce mode de l'invention est inférieur à 10%, de préférence inférieur à 5%, voire inférieur à 3%.

Alternativement, la monodispersité peut être mise en évidence en plaçant un échantillon d'une composition selon l'invention dans un flacon à section circulaire constante. Une agitation douce par rotation de un quart de tour sur une demi-seconde autour de l'axe de symétrie traversant le flacon, suivie d'un repos d'une demi-seconde est effectuée, avant de répéter l'opération en sens inverse, et ce quatre fois de suite.

Les gouttes (G2) s'organisent sous une forme cristalline lorsqu'elles sont monodispersées. Ainsi, elles présentent un empilement suivant un motif se répétant dans les trois dimensions. Il est alors possible d'observer, un empilement régulier qui indique une bonne monodispersité, un empilement irrégulier traduisant la polydispersité de la dispersion.

Avantageusement, une composition selon l'invention peut comprendre de 1% à 50%, de préférence de 3% à 40%, et mieux de 5% à 30%, en poids de gouttes (G1) par rapport au poids total de ladite composition.

Avantageusement, une composition selon l'invention peut comprendre de 0,5% à 40%, en particulier de 1% à 30%, de préférence de 2% à 20%, et mieux de 3% à 15%, voire de 5% à 10%, en poids de gouttes (G2) par rapport au poids total de ladite composition.

Avantageusement, une composition selon l'invention peut comprendre un rapport pondéral « gouttes (G1) / gouttes (G2) » compris entre 0,025 et 100, en particulier entre 0,03 et 50, de préférence entre 0,15 et 20, et mieux entre 0,33 et 10.

***Viscosité***

La viscosité des compositions selon l'invention peut varier de façon importante ce qui permet donc d'obtenir des textures variées.

Selon un mode de réalisation, une composition selon l'invention a une viscosité comprise de 1 mPa.s à 500 000 mPa.s, de préférence de 10 à 300 000 mPa.s, et mieux de 1 000 mPa.s à 100 000 mPa.s, telle que mesurée à 25°C.

Selon un mode de réalisation, la phase aqueuse d'une composition selon l'invention, à température ambiante et pression atmosphérique, a une viscosité comprise de 1 mPa.s à 500 000 mPa.s, de préférence de 10 à 300 000 mPa.s, et mieux de 1 000 mPa.s à 100 000 mPa.s, telle que mesurée à 25°C.

Selon un mode de réalisation, la phase grasse figurée par les gouttes (G1) d'une composition selon l'invention, à température ambiante et pression atmosphérique, a une viscosité comprise de 0,1 mPa.s à 1 000 000 mPa.s, de préférence de 0,5 mPa.s à 500 000 mPa.s, et mieux de 1 mPa.s à 1 000 mPa.s, telle que mesurée à 25°C.

Selon un mode de réalisation, la phase grasse figurée par les gouttes (G2) d'une composition selon l'invention, à température ambiante et pression atmosphérique, a une viscosité comprise de 20 000 mPa.s à 100 000 000 mPa.s, de préférence de 50 000 mPa.s à 1 000 000 mPa.s, et mieux de 100 000 mPa.s à 500 000 mPa.s, à 25°C.

Pour des raisons de faisabilité technique, notamment au vu des procédés de fabrication des compositions selon l'invention tels que décrits ci-après, la viscosité de l'émulsion comprenant les gouttes (G1) est adaptée pour assurer l'homogénéisation, voire la fabrication, des gouttes (G2) dans l'émulsion comprenant les gouttes (G1), et ainsi la fabrication d'une composition selon l'invention.

Au vu des éléments techniques fournis dans la présente description, cette adaptation relève des compétences

de l'homme du métier.

Le cas échéant et si nécessaire, l'homme du métier pourra rehausser la viscosité de la composition selon l'invention, en particulier la viscosité de la phase aqueuse continue, en ajoutant une solution d'augmentation de la viscosité. De préférence, cette solution d'augmentation de la viscosité comprend une base, notamment un hydroxyde d'alcalin, tel que l'hydroxyde de sodium, comme indiqué plus en détails ci-après.

La viscosité est mesurée à température ambiante, par exemple T=25°C ± 2°C et à pression ambiante, par exemple 1 013 mbar, par la méthode décrite ci-après.

On utilise un viscosimètre de type Brookfield, typiquement un viscosimètre numérique Brookfield RVDV-E (couple de torsion du ressort de 7187,0 dyne-cm), qui est un viscosimètre rotationnel à vitesse imposée muni d'un mobile (désigné par le terme anglais « Spindle »). Une vitesse est imposée au mobile en rotation et la mesure du couple exercé sur le mobile permet de déterminer la viscosité en connaissant les paramètres de géométrie/forme du mobile utilisé.

On utilise par exemple un mobile de taille No. 04 (référence Brookfield: RV4). Le taux de cisaillement correspondant à la mesure de la viscosité est défini par le mobile utilisé et la vitesse de rotation de celui-ci.

La mesure de viscosité est effectuée sur 1 minute à température ambiante (T=25°C ± 2°C). On place environ 150 g de solution dans un bécher de 250 ml de volume, ayant un diamètre d'environ 7 cm de façon à ce que la hauteur du volume occupée par les 150 g de solution soit suffisante pour arriver à la jauge marquée sur le mobile. Ensuite, on démarre le viscosimètre sur une vitesse de 10 tours/min et on attend que la valeur affichée sur l'écran soit stable. Cette mesure donne la viscosité du fluide testé, telle que mentionnée dans le cadre de la présente invention.

***Phase grasse***

Comme indiqué ci-dessus, la phase grasse dispersée des compositions de l'invention est un mélange de différentes gouttes (G1) et (G2).

Ces gouttes sont de tailles différentes mais peuvent être de nature identique ou différente en ce qui concerne, notamment, la nature des polymères anioniques et cationiques, voire des huiles et/ou des composés et/ou actifs additionnels décrits ci-après.

*Huile(s)*

Selon un mode de réalisation, la phase grasse des gouttes (G1) et/ou (G2) peut comprendre au moins une huile (H1) dans laquelle les polymères cationiques (PC1) et (PC2) sont solubles. En effet, la composition selon l'invention peut comprendre au moins une huile compatible avec les polymères cationiques (PC1) et (PC2). L'huile (H1) correspond donc à un bon solvant des polymères cationiques (PC1) et (PC2).

Les gouttes (G1) et/ou (G2) selon l'invention peuvent comprendre une seule huile (H1) ou un mélange de plusieurs huiles (H1). Une goutte (G1) et/ou (G2) selon l'invention peut donc comprendre au moins une, au moins deux, au moins trois, au moins quatre, au moins cinq, voire plus, d'huile(s) (H1) telle(s) que décrite(s) ci-après.

On entend par « huile » un corps gras liquide à la température ambiante (25°C).

Selon un mode de réalisation, l'huile (H1) des gouttes (G1) peut être identique ou différente de l'huile (H1) des gouttes (G2). De même, lorsque les gouttes (G1) comprennent un mélange d'huiles (H1), le mélange d'huiles (H1) des gouttes (G2) peut être identique ou différent.

Selon un mode de réalisation, les gouttes (G1) peuvent comprendre une huile (H1) unique et les gouttes (G2) peuvent comprendre un mélange d'huiles (H1), et réciproquement.

Comme huiles (H1) selon l'invention, on peut citer par exemple :

- les huiles hydrocarbonées d'origine animale, telles que le perhydrosqualène et le squalane ;
- les esters et les éthers de synthèse, notamment d'acides gras, comme les huiles de formules $R_1COOR_2$ et $R_1OR_2$ dans laquelle $R_1$ représente le reste d'un acide gras en $C_8$ à $C_{29}$, et $R_2$ représente une chaîne hydrocarbonée, ramifiée ou non, en $C_3$ à $C_{30}$, comme par exemple l'huile de Purcellin, l'isononanoate d'isononyle, le néopentanoate d'isodécyle, le néopentanoate d'isostéaryle, le myristate d'isopropyle, le myristate d'octyldodécyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétrabéhénate de pentaérythrityle (DUB PTB) ou le tétraisostéarate de pentaérythrityle (Prisorine 3631) ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam ;
- les huiles de silicone, comme par exemple les polyméthylsiloxanes (PDMS) volatiles ou non à chaine siliconée

linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes (ou diméthicones) comportant des groupements alkyle, alcoxy ou phényle, pendant ou en bout de chaine siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;

- les alcools gras ayant de 8 à 26 atomes de carbone, comme l'alcool cétylique, l'alcool stéarylique et leur mélange (alcool cétylstéarylique), ou encore l'octyldodécanol ;
- les huiles fluorées partiellement hydrocarbonées et/ou siliconées comme celles décrites dans le document JP-A-2-295912 ;
- et leurs mélanges.

Selon un mode de réalisation, l'huile (H1) est choisie parmi les esters de formule $R_1COOR_2$, dans laquelle $R_1$ représente le reste d'un acide gras en $C_8$ à $C_{29}$, et $R_2$ représente une chaîne hydrocarbonée, ramifiée ou non, en $C_3$ à $C_{30}$.
Selon un mode de réalisation, l'huile (H1) est choisie parmi les alcools gras ayant de 8 à 26 atomes de carbone.
Selon un mode de réalisation, l'huile (H1) est choisie parmi les huiles hydrocarbonées ayant de 8 à 16 atomes de carbone, et notamment les alcanes ramifiés en $C_8$-$C_{16}$ (appelées aussi isoparaffines ou isoalcanes), comme l'isododécane (encore appelé 2,2,4,4,6-pentaméthylheptane), l'isodécane, l'isohexadécane, et, par exemple, les huiles vendues sous les noms commerciaux d'Isopars® ou de Permethyls®.
Selon un mode de réalisation préféré, l'huile (H1) est choisie dans le groupe constitué de l'isononanoate d'isononyle, de la diméthicone, de l'isohexadécane, du polydiméthylsiloxane, de l'octyldodécanol, du néopentanoate d'isodécyle, du néopentanoate d'isostéaryle et de leurs mélanges.
Selon un autre mode de réalisation préféré, l'huile (H1) est choisie parmi les huiles de silicone. De préférence, la phase grasse des gouttes (G1) et/ou (G2) ne comprend pas d'autres huiles différentes des huiles de silicone. De préférence, les huiles présentes dans la phase grasse des gouttes (G1) et/ou (G2) sont des huiles de silicone.
De préférence, l'huile (H1) est l'isononanoate d'isononyle.
Selon un mode de réalisation, l'huile (H1) n'est pas une huile végétale.
Selon un mode de réalisation, l'huile (H1) n'est pas une huile de silicone, et de préférence n'est pas choisie parmi les polydiméthylsiloxanes (PDMS).
Selon une variante de réalisation préférée, l'huile (H1) pouvant être présente dans les gouttes (G2) selon l'invention n'est pas une huile de silicone ou une huile fluorée.
Selon une variante de réalisation préférée, les gouttes (G2) selon l'invention peuvent comprendre au moins une huile de silicone ou une huile fluorée, sous réserve de comprendre en outre au moins une huile (H1) telle que décrite ci-dessus mais différente d'une huile de silicone ou d'une huile fluorée.
Selon un mode de réalisation préféré, les gouttes (G1) et (G2) comprennent de l'isononanoate d'isononyle.
Selon un mode de réalisation préféré, les gouttes (G1) et/ou (G2) selon l'invention comprennent au moins 1% en poids d'huile(s) (H1), de préférence d'isononanoate d'isononyle, par rapport au poids total de ladite composition.
Selon un autre mode de réalisation, une composition selon l'invention, en particulier la phase grasse des gouttes (G1) et/ou (G2), ne comprend pas de polydiméthylsiloxane (PDMS), et de préférence ne comprend pas d'huile de silicone.
Selon un autre mode de réalisation, une composition selon l'invention ne comprend pas d'huile végétale.
Selon un mode de réalisation, la teneur en huile(s) (H1) dans une composition selon l'invention est comprise entre 1% et 99,49 %, de préférence entre 20% et 90%, et en particulier entre 50% et 80%, en poids par rapport au poids total de la phase grasse des gouttes (G1) et/ou (G2).
Selon un mode de réalisation, la phase grasse des gouttes (G1) et/ou (G2) de l'invention peut comprendre en outre au moins une huile hydrocarbonée d'origine végétale (H2). La phase grasse des gouttes (G1) et/ou (G2) peut comprendre plusieurs huiles (H2).
Préférentiellement, on utilise selon l'invention, à titre d'huile(s) (H2), les composés suivants : les triglycérides d'acides caprylique et caprique, les triglycérides d'acides caprylique, caprique, myristique et stéarique (nom INCI : Caprylic/capric/myristic/stearic Triglyceride), le triéthylhexanoine, l'huile de graine de limnanthe *Limnanthes Alba* (nom INCI : Limnanthes Alba (Meadowfoam) Seed Oil), l'huile de noix de macadamia (nom INCI : Macadamia Ternifolia Seed Oil), l'huile d'églantier *Rosa Canina* (nom INCI : Rosa Canina Fruit Oil), l'huile de soja (nom INCI : Glycine Soja (Soybean) Oil), l'huile de graines de tournesol (nom INCI : Helianthus Annuus (Sunflower) Seed Oil), le tribéhénine (nom INCI : tribehenin), le triisostéarine (nom INCI : triisostearin), l'huile de noyau d'abricot (nom INCI : Prunus Armeniaca (Apricot) Kernel Oil), l'huile de son de riz (nom INCI : Oryza Sativa (Rice) Bran Oil), l'huile d'argan (nom INCI : Argania Spinosa Kernel Oil), l'huile d'avocat (nom INCI : Persea Gratissima Oil), l'huile d'onagre (nom INCI : Oenothera Biennis Oil), l'huile de germe de riz (nom INCI : Oryza Sativa Germ Oil), l'huile de noix de coco hydrogénée (nom INCI : Hydrogenated Coconut Oil), l'huile d'amande douce (nom INCI : Prunus Amygdalus Dulcis Oil), l'huile de graine de sésame (nom INCI : Sesamum Indicum Seed Oil), l'huile de colza hydrogénée (nom INCI : Hydrogenated Rapeseed Oil), l'huile de graine

de carthame (nom INCI : Carthamus Tinctorius Seed Oil), l'huile de noix du Queensland *Macadamia integrifolia* (nom INCI : Macadamia Integrifolia Seed Oil), le tricaprylin (ou triacylglycérol), l'huile de germe de blé (nom INCI : Triticum Vulgare Germ Oil), l'huile de graine de bourrache (nom INCI : Borago Officinalis Seed Oil), l'huile de karité (nom INCI : Butyrospermum Parkii Oil), l'huile de ricin hydrogénée (nom INCI : Hydrogenated Castor Oil), l'huile de graine de chou chinois (nom INCI : Brassica Campestris Seed Oil), l'huile de camélia, et notamment de graine de camélia du Japon (nom INCI : Camellia Japonica Seed Oil), l'huile de graine de thé vert (nom INCI : Camellia Sinensis Seed Oil), l'huile d'argousier (nom INCI : Hippophae Rhamnoides Oil), l'huile de graine de *Camellia Kissi* (nom INCI : Camellia Kissi Seed Oil), l'huile de graine de *Moringa* (nom INCI : Moringa Pterygosperma Seed Oil), l'huile de canola (nom INCI : Canola Oil), l'huile de graine de thé (nom INCI : Camellia Oleifera Seed Oil), l'huile de graine de carotte (nom INCI : Daucus Carota Sativa Seed Oil), le triheptanoine (nom INCI : Triheptanoin), l'huile de vanille (nom INCI : Vanilla Planifolia Fruit Oil), les glycérides d'huile de canola et de phytostérols (nom INCI : Phytosteryl Canola Glycerides), l'huile de graine de cassissier (nom INCI: Ribes Nigrum (Black Currant) Seed Oil), l'huile de graine de karanja (nom INCI : Pongamia Glabra Seed Oil), l'huile de roucou (nom INCI : Roucou (Bixa orellana) Oil), et leurs mélanges.

De préférence, l'huile (H2) est choisie parmi les huiles végétales riches en acides gras polyinsaturés.

On entend par "acide gras insaturé" au sens de la présente invention, un acide gras comprenant au moins une double liaison.

Selon un mode de réalisation préféré, on utilise à titre d'huile (H2) des acides gras insaturés comportant de 18 à 22 atomes de carbone, en particulier les acides gras polyinsaturés, notamment les acides gras $\omega$-3 et $\omega$-6.

Parmi les acides gras polyinsaturés de la série $\omega$-6, on peut citer en particulier l'acide linoléique à 18 atomes de carbone et deux insaturations (18:2, $\omega$-6), l'acide $\gamma$-linolénique à 18 atomes de carbone et trois insaturations (18:3, $\omega$-6), l'acide dihomogamalinolénique à 20 atomes de carbone et 3 insaturations (20:3, $\omega$-6), l'acide arachidonique, l'acide 5,8,11,14 éicosatétraénoïque (20:4, $\omega$-6) et l'acide docosatétraénoique (22:4, $\omega$-6).

Les acides gras polyinsaturés de la série $\omega$-3 peuvent notamment être choisis parmi l'acide $\alpha$-linolénique (18:3, $\omega$-3), l'acide stéaridonique (18:4, $\omega$-3), l'acide 5,8,11,14,17-eicosapentaénoïque ou EPA (20:5, $\omega$-3), et l'acide 4,7,10,13,16,19-docosahéxaénoïque ou DHA (22:6, $\omega$-3), l'acide docosapentanoique (22,5, $\omega$-3), l'acide n-butyl-5,11,14-eicosatriénonique.

Selon un mode de réalisation, la teneur en huile(s) (H2) dans la phase grasse des gouttes (G1) et/ou (G2) d'une composition selon l'invention est comprise entre 0% et 40%, de préférence entre 0,1% et 25%, et en particulier entre 1%et 20%, en poids par rapport au poids total de phase grasse de ladite composition.

Selon un mode de réalisation, le ratio massique entre la quantité d'huile(s) (H1) et la quantité d'huile(s) (H2) va de 0,025 à 99,99, de préférence de 0,8 à 90, et en particulier de 2,5 à 80.

La phase grasse peut comprendre en outre au moins une autre huile différente des huiles (H1) et (H2).

Une composition selon l'invention peut comprendre de 0,0001% à 50%, de préférence de 0,1% à 40%, et mieux de 1% à 25%, en poids d'huile(s) par rapport au poids total de ladite composition.

Avantageusement, la phase grasse des gouttes (G2) et/ou (G1) comprend au moins une huile ayant un indice de réfraction proche de celui de la phase continue aqueuse, à savoir une huile ayant un indice de réfraction, à température ambiante (25°C) et pression atmosphérique, compris de préférence entre 1,2 et 1,6, de préférence entre 1,25 et 1,5, en particulier entre 1,3 et 1,4. Ce mode de réalisation est avantageux en ce qu'il permet d'améliorer la transparence de la phase grasse des gouttes (G2) et/ou (G1), et donc la transparence de la composition selon l'invention. Avantageusement, l'huile ayant un indice de réfraction compris entre 1,2 et 1,6 est une huile de silicone, en particulier une huile de silicone phénylée.

***Agent gélifiant***

Comme indiqué ci-dessus, la phase grasse des gouttes (G2) peut en outre comprendre au moins un agent gélifiant. Elle peut donc comprendre un agent gélifiant unique ou un mélange d'agents gélifiants. De préférence, la phase grasse des gouttes (G2) comprend au moins un agent gélifiant.

Un tel agent gélifiant est différent des polymères anioniques et cationiques décrits ci-dessus.

Dans le cadre de l'invention, et sauf mention contraire, on entend par « agent gélifiant », un agent permettant d'augmenter la viscosité de la phase grasse des gouttes (G2) dépourvue dudit agent gélifiant, et d'atteindre une viscosité finale de la phase grasse gélifiée supérieure à 20 000 mPa.s, de préférence supérieure à 50 000 mPa.s, mieux supérieure à 100 000 mPa.s, et tout particulièrement supérieure à 200 000 mPa.s.

Le choix en agent(s) gélifiant(s) s'effectue notamment au regard de la nature de la phase dispersée. Ainsi, pour des raisons de compatibilité, l'agent gélifiant est lipophile.

Selon un mode de réalisation, seules les gouttes (G2) comprennent au moins un agent gélifiant. En d'autres termes, selon ce mode de réalisation, les gouttes (G1) sont dénuées d'agent gélifiant.

Selon un autre mode de réalisation, les gouttes (G2) et (G1) comprennent au moins un agent gélifiant, identique ou différent.

Selon un mode de réalisation, l'agent gélifiant est choisi dans le groupe constitué des agents gélifiants lipophiles organiques, minéraux, polymériques ou moléculaires ; des corps gras solides à température et pression ambiantes, notamment choisis parmi les cires, les corps gras pâteux, les beurres ; et de leurs mélanges.

*Agent(s) gélifiant(s) lipophile(s)*

Les gélifiants utilisables selon l'invention peuvent être des gélifiants lipophiles organiques ou minéraux, polymériques ou moléculaires.

Selon un mode de réalisation, l'agent gélifiant est choisi dans le groupe constitué des argiles modifiées, des silices, telles que la silice pyrogénée, et de leurs mélanges.

Comme gélifiant lipophile minéral, on peut citer les argiles éventuellement modifiées comme les hectorites modifiées par un chlorure d'ammonium en $C_{10}$ à $C_{22}$, comme l'hectorite modifiée par du chlorure de di-stéaryl di-méthyl ammonium telle que, par exemple, celle commercialisée sous la dénomination de Bentone 38V® par la société ELEMENTIS. On peut également citer l'hectorite modifiée par du chlorure de distéaryldiméthylammonium, connue également comme bentonite de quaternium-18, telle que les produits commercialisés ou fabriqués sous les dénominations Bentone 34 par la société Rheox, Claytone XL, Claytone 34 et Claytone 40 commercialisés ou fabriqués par la société Southern Clay, les argiles modifiées connues sous la dénomination de bentonites de benzalkonium et de quaternium-18 et commercialisées ou fabriquées sous les dénominations Claytone HT, Claytone GR et Claytone PS par la société Southern Clay, les argiles modifiées par du chlorure de stéaryldiméthylbenzoylammonium, connues comme bentonites de stéralkonium, telles que les produits commercialisés ou fabriqués sous les dénominations Claytone APA et Claytone AF par la société Southern Clay, et Baragel 24 commercialisé ou fabriqué par la société Rheox.

On peut également citer la silice pyrogénée éventuellement traitée hydrophobe en surface dont la taille des particules est inférieure à 1 $\mu$m. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe.

Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8ème édition, 2000). Elles sont par exemple commercialisées sous les références Aerosil R812® par la société DEGUSSA, CAB-O-SIL TS-530® par la société CABOT ; ou
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8ème édition, 2000). Elles sont par exemple commercialisées sous les références Aerosil R972®, et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200 nm.

On peut également citer les aérogels de silice hydrophobes, de préférence de silice silylée (nom INCI : silica silylate). Concernant la préparation de particules d'aérogels de silice hydrophobe modifiés en surface par silylation, on peut se référer au document US 7,470,725. A titre de d'aérogels de silice hydrophobe utilisables dans l'invention, on peut citer par exemple l'aérogel commercialisé sous la dénomination VM-2260 ou VM-2270 (nom INCI : Silica silylate), par la société Dow Corning. On peut également citer les aérogels commercialisés par la société Cabot sous les références Aerogel TLD 201, Aerogel OGD 201, Aerogel TLD 203, ENOVA® Aerogel MT 1100, ENOVA Aerogel MT 1200.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous

la dénomination Versagel® comme par exemple le mélange de copolymère tribloc butylène/éthylène/styrène et de copolymère étoile éthylène/propylène/styrène dans l'isododécane (Versagel M 5960).

Selon un mode de réalisation, l'agent gélifiant utilisable selon l'invention est choisi dans le groupe constitué des polyacrylates, des esters de dextrine et d'acide(s) gras, des esters de glycérol et d'acide(s) gras, des polyamides, et de leurs mélanges.

Comme gélifiant lipophile, on peut encore citer les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO 02/056847, WO 02/47619, en particulier les résines de polyamides (notamment comprenant des groupes alkyles ayant de 12 à 22 atomes de carbone) telles que celles décrites dans US 5 783 657.

A titre d'exemple de résine de polyamide pouvant être mise en œuvre selon la présente invention, on peut citer UNICLEAR 100 VG® commercialisé par la société ARIZONA CHEMICAL.

On peut également utiliser les polyamides siliconés du type polyorganosiloxane tels que ceux décrits dans US 5 874 069, US 5 919 441, US 6 051 216 et US 5 981 680.

Ces polymères siliconés peuvent appartenir aux deux familles suivantes :

- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

Selon un mode de réalisation, l'agent gélifiant lipophile est un ester de dextrine et d'acide gras, tels que les palmitates de dextrine.

Selon un mode de réalisation, l'ester de dextrine et d'acide(s) gras selon l'invention est un mono- ou poly-ester de dextrine et d'au moins un acide gras répondant à la formule (II) suivante :

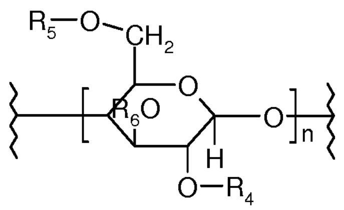

(II)

dans laquelle :

- ◦ n est un nombre entier allant de 2 à 200, de préférence allant de 20 à 150, et en particulier allant de 25 à 50,
- ◦ les radicaux $R_4$, $R_5$ et $R_6$, identiques ou différents, sont choisis parmi l'hydrogène ou un groupement acyle -$COR_a$ dans lequel le radical $R_a$ représente un radical hydrocarboné, linéaire ou ramifié, saturé ou insaturé, possédant de 5 à 50, de préférence de 5 à 25 atomes de carbone,

sous réserve qu'au moins un desdits radicaux $R_4$, $R_5$ ou $R_6$ est différent de l'hydrogène.

Selon un mode de réalisation, $R_4$, $R_5$ et $R_6$ représentent, indépendamment les uns des autres, H ou un groupement acyle -$C(O)R_a$ dans lequel $R_a$ est un radical hydrocarboné tel que défini précédemment, sous réserve qu'au moins deux desdits radicaux $R_4$, $R_5$ ou $R_6$ sont identiques et différents de l'hydrogène.

Selon un mode de réalisation, lorsque les radicaux $R_4$, $R_5$ et $R_6$, identiques ou différents, représentent un radical -$C(O)R_a$, ceux-ci peuvent être choisis parmi les radicaux caprylyle, caproyle, lauroyle, myristyle, palmityle, stéaryle, eicosanyle, docosanoyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécénoyle, palmitoléyle, oléyle, élaidyle, eicosényle, sorbyle, linoléyle, linolényle, punicyle, arachidonyle, stéarolyle, et leurs mélanges.

Parmi les esters de dextrine et d'acide(s) gras, on peut par exemple citer les palmitates de dextrine, les myristates de dextrine, les palmitates/éthylhexanoates de dextrine et leurs mélanges.

On peut notamment citer les esters de dextrine et d'acide(s) gras commercialisés sous les dénominations Rheopearl® KL2 (nom INCI : dextrin palmitate), Rheopearl® TT2 (nom INCI : dextrin palmitate ethylhexanoate), et Rheopearl® MKL2 (nom INCI : dextrin myristate) par la société Miyoshi Europe.

Par rapport au Rheopearl® KL2, le Rheopearl MKL2 (INCI : Dextrin Myristate) est avantageux en ce qu'il conduit à des gouttes (G2), et donc à une composition selon l'invention, présentant une transparence améliorée.

Selon un mode de réalisation, l'agent gélifiant est choisi parmi les polyacrylates résultant de la polymérisation d'acrylate(s) d'alkyle en $C_{10}$-$C_{30}$, de préférence d'acrylate(s) d'alkyle en $C_{14}$-$C_{24}$, et encore plus préférentiellement d'acrylate(s) d'alkyle en $C_{18}$-$C_{22.}$

Selon un mode de réalisation, les polyacrylates sont des polymères d'acide acrylique estérifié avec un alcool gras dont la chaîne carbonée saturée comprend de 10 à 30 atomes de carbone, de préférence de 14 à 24 atomes de carbone, ou un mélange desdits alcools gras. De préférence, l'alcool gras comprend 18 atomes de carbone ou 22 atomes de carbone.

Parmi les polyacrylates, on peut citer plus particulièrement le polyacrylate de stéaryle, le polyacrylate de béhényle. De préférence, l'agent gélifiant est le polyacrylate de stéaryle ou le polyacrylate de béhényle.

On peut notamment citer les polyacrylates commercialisés sous les dénominations Interlimer® (nom INCI : Poly $C_{10}$-$C_{30}$ alkyl acrylate), notamment Interlimer® 13.1 et Interlimer® 13.6, par la société Airproducts.

Selon un mode de réalisation, l'agent gélifiant est un ester de glycérol et d'acide(s) gras, en particulier un mono-, di- ou triester de glycérol et d'acide(s) gras. Typiquement, ledit ester de glycérol et d'acide(s) gras peut être utilisé seul ou en mélange.

Selon l'invention, il peut s'agir d'un ester de glycérol et d'un acide gras ou d'un ester de glycérol et d'un mélange d'acides gras.

Selon un mode de réalisation, l'acide gras est choisi dans le groupe constitué de l'acide béhénique, de l'acide isooctadécanoique, de l'acide stéarique, de l'acide eicosanoïque, et de leurs mélanges.

Selon un mode de réalisation, l'ester de glycérol et d'acide(s) gras présente la formule (I) suivante :

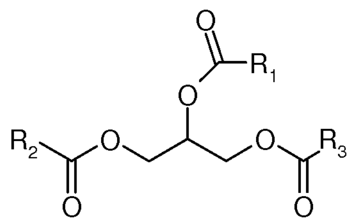

(I)

dans laquelle : $R_1$, $R_2$ et $R_3$ sont, indépendamment l'un de l'autre, choisis parmi H et une chaîne alkyle saturée comprenant de 4 à 30 atomes de carbone, au moins un de $R_1$, $R_2$ et $R_3$ étant différent de H.

Selon un mode de réalisation, $R_1$, $R_2$ et $R_3$ sont différents.

Selon un mode de réalisation, $R_1$, $R_2$ et/ou $R_3$ représente(nt) une chaîne alkyle saturée comprenant de 4 à 30, de préférence de 12 à 22, et préférentiellement de 18 à 22 atomes de carbone.

Selon un mode de réalisation, l'ester de glycérol et d'acide(s) gras correspond à un composé de formule (I) dans laquelle $R_1$ = H, $R_2$ = $C_{21}H_{43}$ et $R_3$ = $C_{19}H_{40}$.

Selon un mode de réalisation, l'ester de glycérol et d'acide(s) gras correspond à un composé de formule (I) dans laquelle $R_1$ = $R_2$ = $R_3$ = $C_{21}H_{43}$.

Selon un mode de réalisation, l'ester de glycérol et d'acide(s) gras correspond à un composé de formule (I) dans laquelle $R_1$ = $R_2$ = H, et $R_3$ = $C_{19}H_{40}$.

Selon un mode de réalisation, l'ester de glycérol et d'acide(s) gras correspond à un composé de formule (I) dans laquelle $R_1$ = $R_2$ = H, et $R_3$ = $C_{17}H_{35}$.

On peut notamment citer les esters de glycérol et d'acide(s) gras commercialisés sous les dénominations Nomcort HK-G (nom INCI : Glyceryl behenate/eicosadioate) et Nomcort SG (nom INCI : Glyceryl tribehenate, isostearate, eicosadioate), par la société Nisshin Oillio.

*Corps gras solides*

Le corps gras solide à température et pression ambiante, différent des agents gélifiants lipophiles décrits précédemment, est notamment choisi dans le groupe constitué des cires, des corps gras pâteux, des beurres et de leurs mélanges.

*Cire(s)*

Par « cire », on entend au sens de l'invention, un composé lipophile, solide à température ambiante (25°C), à changement d'état solide/liquide réversible, ayant un point de fusion supérieur ou égal à 30°C pouvant aller jusqu'à 120°C.

Le protocole de mesure de ce point de fusion est décrit plus loin.

Les cires susceptibles d'être utilisées dans une composition selon l'invention sont choisies parmi les cires, solides, déformables ou non à température ambiante, d'origine animale, végétale, minérale ou de synthèse et leurs mélanges.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac ; la cire de montan, les cires microcristallines, les paraffines et l'ozokérite ; les cires de polyéthylène, les cires obtenues par la synthèse de Fisher-Tropsch, les copolymères cireux ainsi que leurs esters, et leurs mélanges.

On peut aussi citer les cires obtenues par hydrogénation catalytique d'huiles animales ou végétales ayant des chaînes grasses, linéaires ou ramifiées, en $C_3$-$C_{32}$.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.

On peut également utiliser les cires obtenues par transestérification et hydrogénation d'huiles végétales, telles que l'huile de ricin ou d'olive, comme les cires vendues sous les dénominations de Phytowax ricin 16L64® et 22L73® et Phytowax Olive 18L57 par la société SOPHIM. De telles cires sont décrites dans la demande FR 2 792 190.

On peut encore citer les cires commercialisées sous les dénominations Kahlwax®2039 (nom INCI : Candelilla cera) et Kahlwax®6607 (nom INCI : Helianthus Annuus Seed Wax) par la société Kahl Wachsraffinerie, Casid HSA (nom INCI : Hydroxystearic Acid) par la société SACI CFPA, Performa®260 (nom INCI : Synthetic wax) et Performa®103 (nom INCI : Synthetic wax) par la société New Phase, et AJK-CE2046 (nom INCI : Cetearyl alcohol, dibutyl lauroyl glutamide, dibutyl ethylhaxanoyl glutamide) par la société Kokyu Alcohol Kogyo.

On peut aussi utiliser des cires siliconées qui peuvent être avantageusement des polysiloxanes substitués, de préférence à bas point de fusion.

Parmi les cires de silicones commerciales de ce type, on peut citer notamment celles vendues sous les dénominations Abilwax 9800, 9801 ou 9810 (GOLDSCHMIDT), KF910 et KF7002 (SHIN ETSU), ou 176-1118-3 et 176-11481 (GENERAL ELECTRIC).

Les cires de silicone utilisables peuvent également être des alkyl ou alcoxydiméthicones tels que les produits commerciaux suivants : Abilwax 2428, 2434 et 2440 (GOLDSCHMIDT), ou VP 1622 et VP 1621 (WACKER), ainsi que les ($C_{20}$-$C_{60}$) alkyldiméthicones, en particulier les ($C_{30}$-$C_{45}$) alkyldiméthicones comme la cire siliconée vendue sous la dénomination SF-1642 par la société GE-Bayer Silicones.

On peut également utiliser des cires hydrocarbonées modifiées par des groupements siliconés ou fluorés comme par exemple : siliconyl candelilla, siliconyl beeswax et Fluorobeeswax de Koster Keunen.

Les cires peuvent également être choisies parmi les cires fluorées.

*Beurre(s) ou corps gras pâteux*

Par « beurre » (également appelé « corps gras pâteux ») au sens de la présente invention, on entend un composé gras lipophile à changement d'état solide/liquide réversible et comportant à la température de 25°C une fraction liquide et une fraction solide, et à pression atmosphérique (760 mm Hg). En d'autres termes, la température de fusion commençante du composé pâteux peut être inférieure à 25°C. La fraction liquide du composé pâteux mesurée à 25°C peut représenter de 9% à 97% en poids du composé. Cette fraction liquide à 25°C représente de préférence entre 15% et 85%, de préférence encore entre 40% et 85% en poids. De préférence, le ou les beurres présentent une température de fin de fusion inférieure à 60°C. De préférence, le ou les beurres présentent une dureté inférieure ou égale à 6 MPa.

De préférence, les beurres ou corps gras pâteux présentent à l'état solide une organisation cristalline anisotrope, visible par observations aux rayons X.

Au sens de l'invention, la température de fusion correspond à la température du pic le plus endothermique observé en analyse thermique (DSC) telle que décrite dans la norme ISO 11357-3 ; 1999. Le point de fusion d'un pâteux ou d'une cire peut être mesuré à l'aide d'un calorimètre à balayage différentiel (DSC), par exemple le calorimètre vendu sous la dénomination "DSC Q2000" par la société TA Instruments.

Concernant la mesure de la température de fusion et la détermination de la température de fin de fusion, les protocoles de préparation des échantillons et de mesure sont les suivants : Un échantillon de 5 mg de corps gras pâteux (ou beurre) ou de cire préalablement chauffé à 80°C et prélevés sous agitation magnétique à l'aide d'une spatule également chauffée est placé dans une capsule hermétique en aluminium, ou creuset. Deux essais sont réalisés pour s'assurer de la reproductibilité des résultats.

Les mesures sont réalisées sur le calorimètre mentionné ci-dessus. Le four est soumis à un balayage d'azote. Le refroidissement est assuré par l'échangeur thermique RCS 90. L'échantillon est ensuite soumis au protocole suivant

en étant tout d'abord mis en température à 20°C, puis soumis à une première montée en température allant de 20°C à 80°C, à la vitesse de chauffe de 5°C/minute, puis est refroidi de 80°C à -80°C à une vitesse de refroidissement de 5°C/minute et enfin soumis à une deuxième montée en température allant de -80°C à 80°C à une vitesse de chauffe de 5°C/minute. Pendant la deuxième montée en température, on mesure la variation de la différence de puissance absorbée par le creuset vide et par le creuset contenant l'échantillon de beurre en fonction de la température. Le point de fusion du composé est la valeur de la température correspondant au sommet du pic de la courbe représentant la variation de la différence de puissance absorbée en fonction de la température. La température de fin de fusion correspond à la température à laquelle 95% de l'échantillon a fondu.

La fraction liquide en poids du beurre (ou corps gras pâteux) à 25°C est égale au rapport de l'enthalpie de fusion consommée à 25°C sur l'enthalpie de fusion du beurre. L'enthalpie de fusion du beurre ou composé pâteux est l'enthalpie consommée par le composé pour passer de l'état solide à l'état liquide.

Le beurre est dit à l'état solide lorsque l'intégralité de sa masse est sous forme solide cristalline. Le beurre est dit à l'état liquide lorsque l'intégralité de sa masse est sous forme liquide. L'enthalpie de fusion du beurre est égale à l'intégrale de l'ensemble de la courbe de fusion obtenue à l'aide du calorimètre suscité, avec une montée en température de 5°C ou 10°C par minute, selon la norme ISO 11357-3:1999. L'enthalpie de fusion du beurre est la quantité d'énergie nécessaire pour faire passer le composé de l'état solide à l'état liquide. Elle est exprimée en J/g.

L'enthalpie de fusion consommée à 25°C est la quantité d'énergie absorbée par l'échantillon pour passer de l'état solide à l'état qu'il présente à 25°C constitué d'une fraction liquide et d'une fraction solide. La fraction liquide du beurre mesurée à 32°C représente de préférence de 30% à 100 % en poids du composé, de préférence de 50% à 100%, de préférence encore de 60% à 100 % en poids du composé. Lorsque la fraction liquide du beurre mesurée à 32°C est égale à 100%, la température de la fin de la plage de fusion du composé pâteux est inférieure ou égale à 32°C. La fraction liquide du beurre mesurée à 32°C est égale au rapport de l'enthalpie de fusion consommée à 32°C sur l'enthalpie de fusion du beurre. L'enthalpie de fusion consommée à 32°C est calculée de la même façon que l'enthalpie de fusion consommée à 23°C.

Concernant la mesure de la dureté, les protocoles de préparation des échantillons et de mesure sont les suivants : la composition selon l'invention ou le beurre est placé dans un moule de 75 mm de diamètre qui est rempli à environ 75% de sa hauteur. Afin de s'affranchir du passé thermique et de contrôler la cristallisation, le moule est placé à l'étuve programmable Vôtsch VC0018 où il est tout d'abord mis en température à 80°C pendant 60 minutes, puis refroidi de 80°C à 0°C à une vitesse de refroidissement de 5°C/minute, puis laissé à la température stabilisée de 0°C pendant 60 minutes, puis soumis à une montée en température allant de 0°C à 20°C, à une vitesse de chauffe de 5°C/minute, puis laissé à la température stabilisée de 20°C pendant 180 minutes. La mesure de la force de compression est réalisée avec le texturomètre TA/TX2i de Swantech. Le mobile utilisé est choisi selon la texture : - mobile cylindrique en acier de 2 mm de diamètre pour les matières premières très rigides ; - mobile cylindrique en acier de 12 mm de diamètre pour les matières premières peu rigides. La mesure comporte 3 étapes : une 1ère étape après détection automatique de la surface de l'échantillon où le mobile se déplace à la vitesse de mesure de 0,1 mm/s, et pénètre dans la composition selon l'invention ou le beurre à une profondeur de pénétration de 0,3 mm, le logiciel note la valeur de la force maximale atteinte ; une 2ème étape dite de relaxation ou le mobile reste à cette position pendant une seconde et où on note la force après 1 seconde de relaxation ; enfin une 3ème étape dite de retrait ou le mobile revient à sa position initiale à la vitesse de 1 mm/s et on note l'énergie de retrait de la sonde (force négative).

La valeur de la dureté mesurée lors de la première étape correspond à la force de compression maximale mesurée en Newton divisée par la surface du cylindre du texturomètre exprimée en $mm^2$ en contact avec le beurre ou la composition selon l'invention. La valeur de dureté obtenue est exprimée en méga-pascals ou MPa.

Le corps gras pâteux ou beurre peut être choisi parmi les composés synthétiques et les composés d'origine végétale. Un corps gras pâteux peut être obtenu par synthèse à partir de produits de départ d'origine végétale.

Le corps gras pâteux est avantageusement choisi parmi :

- la lanoline et ses dérivés tels que l'alcool de lanoline, les lanolines oxyéthylénées, la lanoline acétylée, les esters de lanoline tels que le lanolate d'isopropyle, les lanolines oxypropylénées,
- les composés siliconés polymères ou non-polymères comme les polydiméthysiloxanes de masses moléculaires élevées, les polydiméthysiloxanes à chaînes latérales du type alkyle ou alcoxy ayant de 8 à 24 atomes de carbone, notamment les stéaryl diméthicones,
- les composés fluorés polymères ou non-polymères,
- les polymères vinyliques, notamment
- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyle ayant de préférence un groupement alkyle en $C_8$-$C_{30}$,

- les oligomères homo et copolymères d'esters vinyliques ayant des groupements alkyles en $C_8$-$C_{30}$,
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,
- les esters et les polyesters, et
- leurs mélanges.

Selon un mode préféré de l'invention, le ou les beurres particuliers sont d'origine végétale tels que ceux décrits dans Ullmann's Encyclopedia of Industrial Chemistry (« Fats and Fatty Oils», A. Thomas, publié le 15/06/2000, D01: 10.1002/14356007.a10_173, point 13.2.2.2. Shea Butter, Borneo Tallow, and Related Fats (Vegetable Butters)).

On peut citer plus particulièrement les triglycérides en $C_{10}$-$C_{18}$ (nom INCI : C10-18 Triglycerides) comportant à la température de 25°C et à pression atmosphérique (760 mm Hg) une fraction liquide et une fraction solide, le beurre de karité, le beurre de Karité Nilotica (*Butyrospermum parkii*), le beurre de Galam, (*Butyrospermum parkii*), le beurre ou graisse de Bornéo ou tengkawang tallow) (*Shorea stenoptera*), le beurre de Shorea, beurre d'Illipé, le beurre de Madhuca ou Bassia Madhuca longifolia, le beurre de mowrah (*Madhuca Latifolia*), le beurre de Katiau (*Madhuca mottleyana*), le beurre de Phulwara (M. *butyracea*), le beurre de mangue (*Mangifera indica*), le beurre de Murumuru (*Astrocatyum murumuru*), le beurre de Kokum (*Garcinia Indica*), le beurre d'Ucuuba (*Virola sebifera*), le beurre de Tucuma, le beurre de Painya (Kpangnan) (*Pentadesma butyracea*), le beurre de café (*Coffea arabica*), le beurre d'abricot (*Prunus Armeniaca*), le beurre de Macadamia (*Macadamia Temifolia*), le beurre de pépins de raisin (*Vitis vinifera*), le beurre d'avocat (*Persea gratissima*), le beurre d'olives (*Olea europaea*), le beurre d'amande douce (*Prunus amygdalus dulcis*), le beurre de cacao (*Theobroma cacao*) et le beurre de tournesol, le beurre sous le nom INCI Astrocaryum Murumuru Seed Butter, le beurre sous le nom INCI Theobroma Grandiflorum Seed Butter, et le beurre sous le nom INCI Irvingia Gabonensis Kernel Butter, les esters de jojoba (mélange de cire et d'huile de jojoba hydrogénée)(nom INCI : Jojoba esters) et les esters éthyliques de beurre de karité (nom INCI : Shea butter ethyl esters), et leurs mélanges.

Selon un mode de réalisation préféré, un agent gélifiant des gouttes (G2), voire des gouttes (G1), selon l'invention est un agent gélifiant thermosensible, à savoir qui réagit à la chaleur, et notamment est un agent gélifiant solide à température ambiante et liquide à une température supérieure à 35°C, de préférence supérieure à 40°C, voire supérieure à 50°C.

De préférence, l'agent gélifiant est choisi parmi les palmitates de dextrine.

Selon un autre mode de réalisation préféré, un agent gélifiant est un agent gélifiant thixotrope ou apte à conférer à la solution qui le comprend un comportement thixotrope. Ce mode de réalisation est avantageux en ce que les gouttes (G2) selon l'invention sont avantageusement obtenues par mise en œuvre d'un procédé microfluidique à température ambiante. Ainsi, l'agent gélifiant est de préférence choisi parmi la silice pyrogénée éventuellement traitée hydrophobe en surface.

Selon un mode de réalisation particulier, une composition selon l'invention, en particulier la phase grasse des gouttes (G2), ne comprend pas de gel d'élastomère comprenant au moins une diméthicone, notamment tel que commercialisé par NuSil Technology sous la dénomination CareSil™ CXG-1104 (INCI : Dimethicone (and) Dimethicone/Vinyl Dimethicone Crosspolymer).

Avantageusement, lorsque la phase grasse des gouttes (G2) comprend au moins un agent gélifiant tel que décrit ci-dessus, en particulier choisi parmi les esters de dextrine et d'acide(s) gras, et de préférence dans le groupe constitué des palmitates de dextrine, des myristates de dextrine, des palmitates/éthylhexanoates de dextrine, et de leurs mélanges, ladite phase grasse des gouttes (G2) comprend en outre au moins une huile ayant un indice de réfraction proche de celui du/des agent(s) gélifiant(s), à savoir une huile ayant un indice de réfraction, à température ambiante (25°C) et pression atmosphérique, compris entre 1,2 et 1,8, de préférence entre 1,3 et 1,7, en particulier entre 1,4 et 1,6, et mieux entre 1,45 et 1,55.

Ce mode de réalisation est avantageux en ce qu'il permet d'améliorer la transparence de la phase grasse des gouttes (G2), et donc la transparence de la composition selon l'invention.

Avantageusement, l'huile ayant un indice de réfraction compris entre 1,2 et 1,8 est une huile de silicone, en particulier une huile de silicone phénylée.

A titre d'huiles de silicone conformes à l'invention, on peut citer par exemple les polyméthylsiloxanes (PDMS) volatiles ou non à chaine siliconée linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes (ou diméthicones) comportant des groupements alkyle, alcoxy ou phényle, pendant ou en bout de chaine siliconée, groupements ayant de 2 à 24 atomes de carbone ; les silicones phénylées comme les phényltriméthicones (en particulier une diphénylsiloxyphényltriméthicone), les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, les polyméthylphénylsiloxanes, et leurs mélanges.

Selon un mode de réalisation, une composition selon l'invention comprend de 0,1% à 75%, de préférence de

0,2% à 60%, en particulier de 0,5% à 40%, mieux de 0,7% à 20%, et préférentiellement de 1% à 4%, en poids d'agent(s) gélifiant(s) par rapport au poids total de la composition.

Selon l'invention, une composition selon l'invention peut comprendre de 0,5% à 98,99%, de préférence de 1% à 70%, en particulier de 1,5% à 50%, mieux de 2% à 40%, en particulier de 2,5% à 30%, et préférentiellement de 10% à 20%, en poids d'agent(s) gélifiant(s) par rapport au poids total de la phase grasse des gouttes (G2), voire des gouttes (G1) et (G2).

De préférence, une composition selon l'invention comprend plus de 10%, de préférence plus de 15%, et mieux plus de 20%, en poids de phase grasse par rapport au poids total de ladite composition.

***Phase aqueuse continue***

Outre les polymères anioniques (PA1) et (PA2) tels que définis ci-dessus, la phase aqueuse des compositions selon l'invention comprend de l'eau, de préférence sous forme d'un gel.

Outre l'eau distillée ou déionisée, une eau convenant à l'invention peut être aussi une eau de source naturelle ou une eau florale.

Selon un mode de réalisation, le pourcentage massique d'eau de la phase continue aqueuse d'une composition selon l'invention est d'au moins 40%, et mieux au moins 50%, notamment compris entre 70% et 98%, préférentiellement compris entre 75% et 95%, par rapport à la masse totale de ladite phase continue.

Les compositions selon l'invention peuvent comprendre au moins 20%, de préférence au moins 30%, en particulier au moins 40%, et mieux au moins 50% en poids d'eau par rapport au poids total de ladite composition.

De préférence, les compositions selon l'invention comprennent au moins 75% en poids de phase aqueuse.

La phase continue aqueuse d'une composition selon l'invention peut en outre comprendre au moins une base. Elle peut comprendre une base unique ou un mélange de plusieurs bases différentes. La présence d'au moins une base dans ladite phase continue aqueuse contribue notamment à rehausser la viscosité de cette dernière.

Selon un mode de réalisation, la base présente dans la phase aqueuse est une base minérale.

Selon un mode de réalisation, la base minérale est choisie dans le groupe constitué des hydroxydes des métaux alcalins et des hydroxydes des métaux alcalino-terreux.

De préférence, la base minérale est un hydroxyde de métaux alcalins, et notamment NaOH.

Selon un mode de réalisation, la base présente dans la phase aqueuse continue est une base organique. Parmi les bases organiques, on peut citer par exemple l'ammoniaque, la pyridine, la triéthanolamine, l'aminométhylpropanol, ou encore la triéthylamine.

Une composition selon l'invention peut comprendre de 0,01 % à 10% en poids, de préférence de 0,01% à 5% en poids, et préférentiellement de 0,02% à 1% en poids de base, de préférence de base minérale, et notamment de NaOH, par rapport au poids total de ladite composition.

***Ecorce des gouttes***

Comme mentionné précédemment, les gouttes (G1) et (G2) selon l'invention sont entourées d'une écorce (également désignée par le terme « membrane »).

Selon l'invention, les gouttes obtenues peuvent présenter une écorce très fine, notamment d'épaisseur inférieure à 1% du diamètre des gouttes (G1) et (G2).

L'épaisseur de l'écorce est ainsi de préférence inférieure à 1 $\mu$m et est trop faible pour être mesurée par des méthodes optiques.

Selon un mode de réalisation, l'épaisseur de l'écorce des gouttes (G1) et (G2) est inférieure à 1 000 nm, notamment comprise de 1 à 500 nm, de préférence inférieure à 100 nm, avantageusement inférieure à 50 nm, préférentiellement inférieure à 10 nm.

La mesure de l'épaisseur de l'écorce des gouttes de l'invention peut être effectuée par la méthode de diffusion de neutrons aux petits angles (Small-Angle X-ray Scattering), telle que mise en œuvre dans Sato et al. J. Chem. Phys. 111, 1393-1401 (2007).

Pour cela, les gouttes sont produites en utilisant de l'eau deutérée, puis sont lavées trois fois avec une huile deutérée, comme par exemple une huile deutérée de type hydrocarboné (octane, dodécane, hexadécane).

Après lavage, les gouttes sont ensuite transférées dans la cellule de Neutrons afin de déterminer le spectre I(q) ; q étant le vecteur d'onde.

A partir de ce spectre, on applique les traitements analytiques classiques (REF) afin de déterminer l'épaisseur de l'écorce hydrogénée (non deutérée).

Selon un mode de réalisation, l'écorce entourant les gouttes de la phase dispersée est rigidifiée, ce qui confère notamment une bonne résistance aux gouttes et diminue, voire empêche, leur coalescence.

Cette écorce est typiquement formée par coacervation, c'est-à-dire par précipitation de polymères chargés de

charges opposées. Au sein d'un coacervat, les liaisons liant les polymères chargés entre eux sont de type ionique, voire de type hydrogène et hydrophobe, et sont généralement plus fortes que des liaisons présentes au sein d'une membrane de type tensioactif.

L'écorce est formée par coacervation d'au moins deux polymères chargés de polarité opposée (ou polyélectrolyte) et de préférence en présence d'un premier polymère, de type cationique, et d'un deuxième polymère, différent du premier polymère, de type anionique. Ces deux polymères jouent le rôle d'agents de stabilisation, voire de rigidification, de la membrane.

La formation du coacervat entre ces deux polymères est généralement provoquée par une modification des conditions du milieu réactionnel (température, pH, concentration en réactifs, etc.). La réaction de coacervation résulte de la neutralisation de ces deux polymères chargés de polarités opposées et permet la formation d'une structure membranaire par interactions électrostatiques entre le polymère anionique et le polymère cationique. La membrane ainsi formée autour de chaque goutte forme typiquement une écorce qui encapsule totalement le cœur de la goutte et isole ainsi le cœur de la goutte de la phase aqueuse continue.

***Polymères anioniques***

Comme indiqué précédemment, les gouttes (G1) et (G2) comprennent des écorces formées d'au moins un polymère anionique (PA1) ou (PA2) respectivement.

Selon l'invention, les polymères anioniques (PA1) et (PA2) sont identiques ou différents.

Les polymères anioniques (PA1) et (PA2) servent à la formation, respectivement, de l'écorce des gouttes (G1) et (G2). Ledit polymère anionique (PA1), voire (PA2), contribue en outre à augmenter la viscosité de la phase continue aqueuse d'une composition selon l'invention.

Dans le cadre de la présente description, on entend par "polymère anionique" (ou "polymère de type anionique") un polymère comportant des fonctions chimiques de type anionique. On peut aussi parler de polyélectrolyte anionique.

Par "fonction chimique de type anionique", on entend une fonction chimique AH capable de céder un proton pour donner une fonction $A^-$. Selon les conditions du milieu dans lequel il se trouve, le polymère de type anionique comporte donc des fonctions chimiques sous forme AH, ou bien sous forme de sa base conjuguée $A^-$.

Comme exemple de fonctions chimiques de type anionique, on peut citer les fonctions acides carboxyliques -COOH, éventuellement présentes sous forme d'anion carboxylate $-COO^-$.

Comme exemple de polymère de type anionique, on peut citer tout polymère formé par la polymérisation de monomères dont au moins une partie porte des fonctions chimiques de type anionique, tel que des fonctions acide carboxylique. De tels monomères sont par exemple l'acide acrylique, l'acide maléique, ou tout monomère éthyléniquement insaturé comportant au moins une fonction acide carboxylique.

De préférence, le polymère anionique est hydrophile, c'est-à-dire soluble ou dispersible dans l'eau. Dans le cadre de l'invention, et sauf mention contraire, on entend par « hydrophile », la propriété selon laquelle un corps donné est compatible avec de l'eau ou un solvant polaire, c'est-à-dire peut accepter de l'eau ou ledit solvant, pour former avec eux une phase homogène, par exemple une solution.

Selon l'invention, les polymères anioniques (PA1) et (PA2) sont identiques ou différents.

Selon un mode de réalisation, les polymères anioniques (PA1) et (PA2), identiques ou différents, sont des polymères comprenant des unités monomériques comportant au moins une fonction acide carboxylique.

Parmi les exemples de polymères anioniques appropriés à la mise en œuvre de l'invention, on peut citer les copolymères d'acide acrylique ou d'acide maléique et d'autres monomères, tels que l'acrylamide, les acrylates d'alkyle, les acrylates d'alkyle en $C_5$-$C_8$, les acrylates d'alkyle en $C_{10}$-$C_{30}$, les méthacrylates d'alkyle en $C_{12}$-$C_{22}$, les méthacrylates méthoxypolyéthylèneglycol, les acrylates d'hydroxyester, les acrylates crosspolymères, et leurs mélanges.

De préférence, les polymères anioniques (PA1) et (PA2), identiques ou différents, sont choisis parmi les carbomères et les copolymères réticulés acrylates/$C_{10}$-$_{30}$ alkyl acrylate. De préférence, les polymères anioniques (PA1) et (PA2) selon l'invention sont des carbomères, identiques ou différents.

Dans le cadre de l'invention, et sauf mention contraire, on entend par "carbomère", un homopolymère éventuellement réticulé, issu de la polymérisation de l'acide acrylique. Il s'agit donc d'un poly(acide acrylique) éventuellement réticulé.

Parmi les carbomères de l'invention, on peut citer ceux commercialisés sous les noms Tego®Carbomer 340FD de Evonik, Carbopol® 981 de Lubrizol, Carbopol ETD 2050 de Lubrizol, ou encore Carbopol Ultrez 10 de Lubrizol.

Selon un mode de réalisation, on entend par "carbomère" ou "carbomer" ou "Carbopol®" un polymère d'acide acrylique de haut poids moléculaire réticulé avec du sucrose allylique ou des éthers allyliques de pentaérythritol (handbook of Pharmaceutical Excipients, 5eme Edition, pIII). Par exemple, il s'agit du Carbopol ®10, du Carbopol®934, du Carbopol®934P, du Carbopol®940, du Carbopol®941, du Carbopol®71G, du Carbopol®980, du Carbopol®971P, ou du Carbopol®974P. Selon un mode de réalisation, la viscosité dudit carbomère est comprise entre 4 000 et 60 000 cP à 0,5% w/w à un pH compris entre 4,5 et 7,0.

Les carbomères ont d'autres dénominations : acides polyacryliques, polymères carboxyvinyliques ou carboxy polyéthylènes.

Selon l'invention, la composition susmentionnée peut comprendre de 0,01 % à 10%, de préférence de 0,05% à 5%, en particulier de 0,1% à 3%, en poids de polymère(s) anionique(s), notamment de carbomère(s), par rapport au poids total de ladite composition.

Selon l'invention, les polymères anioniques (PA1) et (PA2) peuvent également être un copolymère réticulé acrylates/$C_{10}$-$_{30}$ alkyl acrylate (nom INCI : acrylates/$C_{10}$-$_{30}$ alkyl acrylate Crosspolymer) tel que défini ci-dessus.

Selon l'invention, les compositions selon l'invention peuvent comprendre un carbomère et un copolymère réticulé acrylates/$C_{10}$-$_{30}$ alkyl acrylate.

***Polymères cationiques***

Comme indiqué précédemment, les gouttes (G1) et (G2) comprennent des écorces formées d'au moins un polymère cationique (PC1) ou (PC2) respectivement.

Selon l'invention, les polymères cationiques (PC1) et (PC2) sont identiques ou différents.

Les gouttes (G1) ou (G2) peuvent également comprendre plusieurs polymères de type cationique.

Dans le cadre de la présente demande, et sauf mention contraire, on entend par "polymère cationique" (ou "polymère de type cationique") un polymère comportant des fonctions chimiques de type cationique. On peut aussi parler de polyélectrolyte cationique.

De préférence, le(s) polymère(s) cationique(s) est/sont lipophile(s) ou liposoluble(s).

Dans le cadre de la présente demande, et sauf mention contraire, par "fonction chimique de type cationique", on entend une fonction chimique B capable de capter un proton pour donner une fonction $BH^+$. Selon les conditions du milieu dans lequel il se trouve, le polymère de type cationique comporte donc des fonctions chimiques sous forme B, ou bien sous forme $BH^+$, son acide conjugué.

Comme exemple de fonctions chimiques de type cationique, on peut citer les fonctions amine primaire, secondaire et tertiaire, éventuellement présentes sous forme de cations ammoniums.

Comme exemple de polymères cationiques, on peut citer tout polymère formé par la polymérisation de monomères dont au moins une partie porte des fonctions chimiques de type cationique, tel que des fonctions amine primaire, secondaire ou tertiaire.

De tels monomères sont par exemple l'aziridine, ou tout monomère éthyléniquement insaturé comportant au moins une fonction amine primaire, secondaire ou tertiaire.

Parmi les exemples de polymères cationiques appropriés à la mise en œuvre de l'invention, on peut citer l'amodiméthicone, dérivé d'un polymère silicone (polydiméthylsiloxane, aussi appelé diméthicone), modifié par des fonctions amine primaire et amine secondaire.

On peut également citer des dérivés de l'amodiméthicone, comme par exemple des copolymères de l'amodiméthicone, l'aminopropyl diméthicone, et plus généralement des polymères silicones linéaires ou ramifiés comportant des fonctions amines.

On peut citer le copolymère de bis-isobutyl PEG-14/amodiméthicone, le Bis (C13-15 Alkoxy) PG-Amodimethicone, le Bis-Cetearyl Amodimethicone et le bis-hydroxy/méthoxy amodiméthicone.

On peut également citer les polymères de type polysaccharide comprenant des fonctions amine, tel que le chitosan ou les dérivés de gomme guar (chlorure d'hydroxypropyltrimonium guar).

On peut également citer les polymères de type polypeptide comprenant des fonctions amine, tel que la polylysine.

On peut également citer les polymères de type polyéthylèneimine comprenant des fonctions amine, tel que la polyéthylèneimine linéaire ou branchée.

Selon un mode de réalisation, les gouttes (G1) ou (G2), et notamment l'écorce desdites gouttes, comprennent un polymère cationique (PC1), ou (PC2) respectivement, qui est un polymère silicone modifié par une fonction amine primaire, secondaire ou tertiaire, tel que l'amodiméthicone.

Selon un mode de réalisation, les gouttes (G1) ou (G2), et en particulier l'écorce desdites gouttes, comprennent de l'amodiméthicone.

Selon un mode de réalisation particulièrement préféré, le polymère cationique répond à la formule suivante :

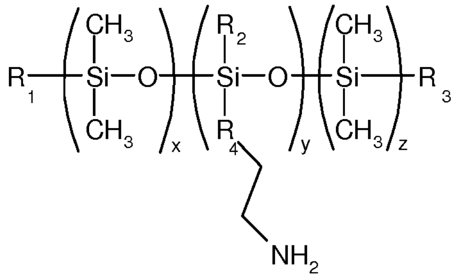

dans laquelle :

- $R_1$, $R_2$ et $R_3$, indépendamment les uns des autres, représentent OH ou $CH_3$ ;
- $R_4$ représente un groupe $-CH_2-$ ou un groupe -X-NH- dans lequel X est un radical alkylène divalent en C3 ou C4 ;
- x est un nombre entier compris entre 10 et 5 000, de préférence entre 30 et 1 000, et mieux entre 80 et 300 ;
- y est un nombre entier compris entre 2 et 1 000, de préférence entre 4 et 100, et mieux entre 5 et 20 ; et
- z est un nombre entier compris entre 0 et 10, de préférence entre 0 et 1, et mieux est égal à 1.

Dans la formule susmentionnée, lorsque $R_4$ représente un groupe -X-NH-, X est relié à l'atome de silicium.

Dans la formule susmentionnée, $R_1$, $R_2$ et $R_3$ représentent de préférence $CH_3$.

Dans la formule susmentionnée, $R_4$ est de préférence un groupe $-(CH_2)_3-NH-$.

Selon l'invention, les gouttes (G1) et (G2) peuvent comprendre de 0,01% à 10%, de préférence de 0,05% à 5%, en particulier de 0,1 à 3%, en poids de polymère(s) cationique(s) (PC1) et (PC2), notamment d'amodiméthicone(s), par rapport au poids total de la phase grasse des gouttes (G1) et (G2).

***Composé(s) / actif(s) additionnel(s)***

Une composition selon l'invention peut en outre comprendre au moins un composé additionnel différent des polymères anioniques et cationiques, des agents gélifiants et des huiles susmentionnés, de préférence choisi parmi des poudres, des paillettes, des agents colorants, notamment choisis parmi les agents colorants hydrosolubles ou non, liposolubles ou non, organiques ou inorganiques, les matériaux à effet optique, les cristaux liquides, des agents particulaires, des élastomères de silicone émulsionnants et/ou non émulsionnants, notamment tels que décrit dans EP2353577, des conservateurs, des humectants, des stabilisateurs, des chélateurs, des agents de texture, des émollients etc... ou tout additif cosmétique usuel, et leurs mélanges.

En particulier, une composition selon l'invention peut en outre comprendre à titre de composé(s) additionnel(s) au moins un adjuvant, tel qu'au moins un composé choisi parmi les charges, et en particulier les poudres à effet soft-focus, les poudres matifiantes, qui peuvent être notamment choisies parmi les polyamides, la silice, le talc, le mica, les fibres (notamment de polyamide ou de cellulose); le nylon-6, le methyl methacrylate crosspolymer, et leurs mélanges.

De tels composés additionnels peuvent être localisés dans la phase aqueuse continue d'une composition selon l'invention, dans les gouttes (G1) et/ou dans les gouttes (G2).

En particulier, une composition selon l'invention, de préférence les gouttes (G2), comprend/comprennent au moins un agent colorant. La présence d'un tel agent colorant permet de disposer de produits cosmétiques avec une attractivité visuelle exacerbée.

Un agent colorant pouvant être utilisé dans le cadre de la présente invention est notamment tel que décrit dans la demande FR1558848.

En particulier, une composition selon l'invention, de préférence la phase continue aqueuse, comprend au moins un agent de texture. La présence d'un tel agent de texture permet de faire varier la fluidité et/ou la sensorialité de la composition. Un agent de texture, notamment hydrophile, c'est-à-dire soluble ou dispersible dans l'eau, pouvant être utilisé dans le cadre de la présente invention, est notamment tel que décrit dans la demande FR1558849.

De manière préférée, les agents de texture de la phase aqueuse sont choisis parmi ceux qui sont résistants aux électrolytes et utilisables sur une large gamme de pH, et sont notamment choisis parmi la méthylcellulose ; l'éthylcellulose ; les copolymères AMPS Na/hydroxyéthyl acrylate commercialisés sous les dénominations Sepinov WEP ou Sepinov EMT 10 ; les acryloyl Dimethyltaurate/Sodium Acrylate/Dimethylacrylamide crosspolymers ; le polyacrylate crosspolymer-6 ; le polyurethane-39 ; le cétyl hydroxyéthylcellulose ; la glycérine ; les carbomères figurés par ceux commercialisés sous les dénominations Carbopol Ultrez 10/30, les copolymères acrylates, notamment ceux commercialisés sous les dénominations Carbopol Aqua SF-1 Polymer ou Carbopol Aqua SF-1 OS Polymer ; les sodium acrylates/beheneth-25 méthacrylate crosspolymer ; les acrylates/acrylamide copolymers ; l'alcasealan (INCI : Alcaligenes Polysaccharides) ; et leurs mélanges, et mieux sont choisis parmi les copolymères acrylates, en particulier celui commercialisé sous la dénomination Carbopol Aqua SF-1 Polymer.

Ces agents de textures, outre leur(s) propriété(s) de résistance aux électrolytes et/ou de stabilité sur une large gamme de pH (en particulier pH entre 4,5 et 7,0), confèrent à une composition selon l'invention les comprenant une stabilité et une transparence améliorée.

Une composition selon l'invention peut encore en outre comprendre au moins un actif additionnel, de préférence choisi parmi les agents hydratants, les agents cicatrisants, les agents dépigmentants, les filtres UV, les agents desquamants, les agents antioxydants, les actifs stimulant la synthèse des macromoléculaires dermiques et/ou épidermiques, les agents dermodécontractants, les agents anti-transpirants, les agents apaisants, les agents anti-âge, les agents parfumants et leurs mélanges. De tels actifs sont notamment décrits dans FR 1 558 849.

A titre représentatif d'actifs utilisables dans la présente invention, on peut plus particulièrement mentionner l'adénosine, le rétinol et ses dérivés, l'acide ascorbique et ses dérivés, tels que le glucoside d'ascorbyle ; le tocophérol et ses dérivés, tels que l'acétate de tocophéryle ; l'acide nicotinique et ses précurseurs, tels que la nicotinamide, les extraits de plantes et notamment les extraits de graines de seigle sous la dénomination commerciale Coheliss de Silab ; un extrait de feuilles de centella asiatica; les extraits huileux ou aqueux de fleurs et/ou de feuilles de camellia japonica; les cellules végétales , un extrait de licorice (glycyrrhiza glabra), un extrait de racines de zingiber, la glycérine ; la diglycérine ; les glycols, tel que le sorbitol; les bétaïnes ; l'urée et ses dérivés, les $\alpha$-hydroxyacides ; les $\beta$-hydroxyacides, tels que l'acide salicylique, les lipopeptides commercialisés par la société SEDERMA sous les dénominations commerciales Biopeptide CL, Matrixyl 500 et Matrixyl 3000, les homo- et co-polymères de l'acide 2-méthacryloyloxyéthylphosphorylcholine, comme Lipidure HM et Lipidure PBM de NOF, les glycosaminoglycanes et leurs dérivés tels que l'acide hyaluronique, le hyaluronate de sodium et l'acide hyaluronique acétylé, les acides aminés libres et leurs dérivés; le glucosamine; l'acide citrique ; les céramides ; et leurs mélanges.

Les compositions peuvent en outre comprendre au moins un composé riche en acides gras polyinsaturés différent d'une huile (H2) telle que décrite ci-dessus et notamment choisi parmi un extrait de microalgues (par exemple spiruline (*Spirulina maxima* et *Spirulina platensis*)), un extrait de zooplancton, une huile de poissons ; et leurs mélanges.

De tels actifs additionnels peuvent être localisés dans la phase aqueuse continue d'une composition selon l'invention, dans les gouttes (G1) et/ou dans les gouttes (G2).

En particulier, une composition selon l'invention, de préférence les gouttes (G1), comprend/comprennent au moins un agent parfumant.

Bien entendu, l'homme du métier veillera à choisir les éventuels composé(s) et/ou actif(s) additionnel(s) et/ou leur quantité de telle manière que les propriétés avantageuses d'une composition selon l'invention, notamment l'intégrité des gouttes (G1) et (G2), ne soient pas ou substantiellement pas altérées par l'adjonction envisagée. Par ailleurs, la nature et/ou la quantité des éventuels composé(s) et/ou actif(s) additionnel(s) dépend(ent) de la nature aqueuse ou grasse de la phase considérée de la composition selon l'invention. Ces ajustements relèvent des compétences de l'homme du métier.

Selon un mode de réalisation, les compositions selon l'invention contiennent moins de 2%, voire moins de 1%, de préférence moins de 0,5%, en poids de composés ayant des propriétés tensioactives.

Préférentiellement, les compositions selon l'invention ne comprennent pas de tensioactif.

Ainsi, la teneur faible en, voire l'absence de, tensioactifs va avantageusement bloquer le phénomène de mûrissement compositionnel des gouttes (G2) vers les gouttes (G1) ce qui permet de garantir le maintien de l'intégrité et des propriétés des gouttes (G2), et donc les propriétés avantageuses d'une composition selon l'invention décrites précédemment.

Au vu de ce qui précède, la phase grasse dispersée d'une composition selon l'invention peut en outre comprendre des gouttes gélifiées (G3), voire des gouttes gélifiées (G4), (G5), etc, mais différentes des gouttes gélifiées (G2).

Ces différences peuvent se réaliser au niveau du diamètre et/ou de la couleur et/ou du polymère cationique et/ou du polymère anionique et/ou de l'agent gélifiant et/ou du/des composé(s) et/ou actif(s) additionnel(s) mis en œuvre pour la préparation des gouttes gélifiées (G2) et (G3), voire des gouttes gélifiées (G4), (G5), etc.

La présente invention concerne également l'utilisation d'une émulsion huile-dans-eau comprenant une phase aqueuse continue et une phase grasse dispersée sous forme de gouttes (G1) telles que définies ci-dessus, pour améliorer la stabilité et/ou préserver l'intégrité de gouttes (G2) telles que définies ci-dessus.

***Procédé de préparation***

Une composition selon l'invention peut être préparée, notamment, via les deux procédés décrits ci-après, respectivement désignés procédé n°1 et procédé n°2.

*Procédé n° 1*

Selon un premier mode de réalisation, la présente invention concerne un procédé de préparation d'une composition telle que définie ci-dessus, comprenant les étapes suivantes :

a) la préparation d'une émulsion (E1) huile-dans-eau comprenant des gouttes (G1), par mélange sous agitation d'une phase grasse dans une phase aqueuse, ladite phase grasse comprenant au moins un polymère cationique (PC1) et en outre, de façon optionnelle, au moins un actif(s) et/ou composé(s) additionnel(s) tel(s) que susmentionné(s) et ladite phase aqueuse comprenant au moins de l'eau et au moins un polymère anionique (PA1) et en outre, de façon optionnelle, au moins un actif(s) et/ou composé(s) additionnel(s) tel(s) que susmentionné(s), voire une base, des conservateurs et/ou d'autres produits solubles dans l'eau tels que la glycérine,
b) la préparation d'une émulsion (E2) huile-dans-eau comprenant une phase aqueuse, identique ou différente à la phase aqueuse de l'émulsion (E1), et des gouttes (G2), par mise en contact d'un fluide huileux FI, ledit fluide huileux FI comprenant au moins un polymère cationique (PC2), identique ou différent de (PC1), et optionnellement au moins un agent gélifiant, et d'un fluide aqueux FE, ledit fluide aqueux FE comprenant au moins de l'eau et au moins un polymère anionique (PA2), identique ou différent de (PA1), pour former des gouttes (G2), de préférence monodisperses, comprenant une phase grasse, constituée du fluide huileux FI, dispersées dans une phase aqueuse continue, constituée de fluide FE, lesdites gouttes (G2) comprenant une écorce isolant le cœur des gouttes de ladite phase grasse,
c) le mélange des émulsions (E1) et (E2), et
d) optionnellement, l'ajout d'une solution d'augmentation de la viscosité des phases aqueuses, de préférence comprenant une base, notamment un hydroxyde d'alcalin, tel que l'hydroxyde de sodium, et/ou d'une solution comprenant au moins un agent de texture des phases aqueuses, notamment tel que décrit précédemment, pour faire varier leur fluidité et/ou sensorialité et donc la fluidité et/ou la sensorialité de la composition selon l'invention.

L'étape b) relative à la préparation de l'émulsion (E2), notamment lorsque le fluide FI comprend au moins un agent gélifiant, en particulier thermosensible, peut consister à :

- chauffer un fluide huileux FI, à une température comprise de 35°C à 150°C, ledit fluide huileux FI comprenant au moins un polymère cationique (PC2), identique ou différent de (PC1), et optionnellement au moins un agent gélifiant et en outre, de façon optionnelle, au moins un composé(s) et/ou actif(s) additionnel(s) tel(s) que susmentionné(s) ; et
- mettre en contact ledit fluide huileux FI et un fluide aqueux FE, ledit fluide aqueux FE comprenant au moins de l'eau et au moins un polymère anionique (PA2), identique ou différent de (PA1), et en outre, de façon optionnelle, au moins un actif(s) et/ou composé(s) additionnel(s) tel(s) que susmentionné(s), voire une base, des conservateurs et/ou d'autres produits solubles dans l'eau tels que la glycérine, pour former des gouttes (G2), de préférence monodisperses, comprenant une phase grasse, constituée du fluide huileux FI, dispersées dans une phase aqueuse continue, constituée de fluide FE, lesdites gouttes (G2) comprenant une écorce isolant le cœur des gouttes de ladite phase grasse.

La température de l'étape de chauffage du fluide FI susmentionnée est adaptée, notamment, à la quantité et/ou la nature de l'agent gélifiant mis en œuvre, en particulier de telle manière qu'il se présente sous forme liquide. Cette adaptation relève des compétences de l'homme du métier.
Dans ce procédé, les étapes a) et b) peuvent être réalisées dans l'ordre a) puis b), b) puis a), voire même en parallèle.
Ce premier mode de réalisation consiste donc à préparer séparément chacune des émulsions (E1) et (E2) et à les mélanger ensuite pour obtenir la composition selon l'invention.
Selon ce premier mode de réalisation, le procédé peut comprendre en outre une étape e), antérieure à l'étape c) mais postérieure à l'étape b), consistant à réaliser une filtration partielle ou totale de l'émulsion (E2) pour éliminer tout ou partie de la phase aqueuse continue (i.e. fluide aqueux FE) et récupérer une solution plus ou moins concentrée en gouttes (G2). Cette étape optionnelle permet alors de contrôler la teneur en gouttes (G2) dans la composition finale.
L'homme du métier saura ajuster le(s) paramètres et/ou matériels requis pour parvenir à la teneur en gouttes (G2) recherchée.
Selon l'invention, cette étape e) de filtration peut être effectuée par filtration mécanique, tamis-passoire, aspiration de la phase aqueuse en continu ou après crémage des gouttes (G2).

*Préparation de l'émulsion (E1) avec le procédé n° 1*

Selon une première variante de réalisation, l'émulsion (E1) susmentionnée est préparée selon un procédé simple « non-microfluidique », à savoir par simple émulsification. Comme dans une émulsion classique, une solution aqueuse et une solution grasse sont préparées séparément. C'est l'ajout sous agitation de la phase grasse dans la phase aqueuse qui crée l'émulsion directe.

Selon une autre variante de réalisation, l'émulsion (E1) susmentionnée est préparée selon un procédé « microfluidique », notamment tel que décrit dans les demandes WO2012/120043 ou WO2015/055748 ou la demande FR1558850.

*Préparation de l'émulsion (E2) avec le procédé n° 1*

L'émulsion (E2) peut être préparée selon un procédé microfluidique, notamment comme décrit dans les demandes WO2012/120043 ou WO2015/055748 ou la demande FR1558850. En d'autres termes, l'émulsion (E2) n'est avantageusement pas préparée selon un procédé « non-microfluidique », à savoir par simple émulsification.

C'est ce procédé microfluidique qui permet de conférer aux gouttes (G2) les propriétés de taille uniforme et de monodispersité décrites précédemment.

Comme indiqué précédemment, les solutions (ou fluides) utilisées pour constituer la phase aqueuse continue et la phase grasse dispersée de cette émulsion (E2) sont respectivement désignées Fluide Externe (FE) et Fluide Interne (FI).

Au vu de ce qui précède, le procédé de préparation de l'émulsion (E2) selon l'invention peut comprendre une étape de chauffage du fluide huileux FI, comprenant la phase grasse de l'émulsion (E2), à une température comprise de 40°C et 150°C, de préférence de 50°C à 90°C, préalablement à l'étape susmentionnée de formation des gouttes (G2), et donc avant mélange/mise en contact de ladite phase grasse avec la phase aqueuse.

Selon un mode de réalisation, la température de l'étape de chauffage est comprise de 50°C à 80°C.

Avantageusement, la présence d'un agent gélifiant dans le fluide huileux FI permet de s'affranchir de l'utilisation d'un fluide intermédiaire tel que décrit dans la demande WO2012/120043.

Avantageusement, le fluide FE est également chauffé à une température comprise de 35°C à 150°C, de préférence de 50°C à 100°C, lors de l'étape b).

*<u>Procédé n°2</u>*

Selon un deuxième mode de réalisation, la présente invention concerne un procédé de préparation d'une composition telle que définie ci-dessus, comprenant les étapes suivantes :

a) la préparation d'une émulsion (E1) huile-dans-eau comprenant des gouttes (G1), par mélange sous agitation d'une phase grasse dans une phase aqueuse, ladite phase grasse comprenant au moins un polymère cationique (PC1), et en outre, de façon optionnelle, au moins un actif(s) et/ou composé(s) additionnel(s) tel(s) que susmentionné(s) et ladite phase aqueuse comprenant au moins de l'eau et au moins un polymère anionique (PA1) et en outre, de façon optionnelle, au moins un actif(s) et/ou composé(s) additionnel(s) tel(s) que susmentionné(s), voire une base, des conservateurs et/ou d'autres produits solubles dans l'eau tels que la glycérine,

b) la mise en contact de l'émulsion (E1) et d'un fluide huileux FI comprenant au moins un polymère cationique (PC2), identique ou différent de (PC1), et optionnellement au moins un agent gélifiant, et la formation des gouttes (G2) comprenant une phase grasse, constituée du fluide huileux FI, dispersées dans l'émulsion (E1) ; et

d) optionnellement, l'ajout d'une solution d'augmentation de la viscosité de la phase aqueuse, de préférence comprenant une base, notamment un hydroxyde d'alcalin, tel que l'hydroxyde de sodium, et/ou d'une solution comprenant au moins un agent de texture, notamment tel que décrit précédemment, pour faire varier la fluidité et/ou la sensorialité de la phase aqueuse et donc de la composition selon l'invention.

L'étape b), notamment lorsque le fluide FI comprend au moins un agent gélifiant, en particulier thermosensible, peut consister à :

- le chauffage d'un fluide huileux FI, à une température comprise de 35°C à 150°C, ledit fluide huileux FI comprenant au moins un polymère cationique (PC2), identique ou différent de PC(1), et au moins un agent gélifiant et en outre, de façon optionnelle, au moins un actif(s) et/ou composé(s) additionnel(s) tel(s) que susmentionné(s) ; et
- la mise en contact de l'émulsion (E1) et dudit fluide huileux FI, et la formation des gouttes (G2) comprenant une phase grasse, constituée du fluide huileux FI, dispersées dans l'émulsion (E1).

La température de l'étape b) de chauffage du fluide FI susmentionnée est adaptée, notamment, à la quantité et/ou la nature de l'agent gélifiant mis en œuvre, en particulier de telle manière qu'il se présente sous forme liquide. Cette adaptation relève des compétences de l'homme du métier.

Avantageusement, l'émulsion (E1) est également chauffée à une température comprise de 35°C à 150°C, de préférence de 50°C à 100°C, lors de l'étape b).

*Préparation de l'émulsion (E1) avec le procédé n°2*

L'émulsion (E1) susmentionnée est préparée selon les mêmes procédés « non-microfluidique » et « microfluidique » que décrits ci-dessus pour le procédé n°1.
Néanmoins, l'émulsion (E1) a ici de préférence une viscosité inférieure à 3000 cPs telle que mesurée à 25°C et selon le protocole décrit précédemment et/ou est chauffée à une température comprise de 35°C à 150°C, de préférence de 50°C à 100°C, lors de l'étape b).

*Préparation de la composition selon l'invention avec le procédé n°2*

Ce deuxième mode de réalisation diffère du premier mode de réalisation ci-dessus en ce qu'une émulsion (E2) n'est pas réalisée. En effet, avec ce procédé n°2, une composition selon l'invention est préparée selon un procédé microfluidique, le cas échéant à chaud, notamment comme décrit dans la demande FR1558850, dans lequel l'émulsion (E1) est utilisée à titre de phase continue (ou fluide FE) pour la fabrication des gouttes (G2).
C'est ce procédé microfluidique qui permet de conférer aux gouttes (G2) les propriétés de taille uniforme et de monodispersité décrites précédemment.
Avantageusement, la présence d'un agent gélifiant dans le fluide huileux FI permet de s'affranchir de l'utilisation d'un fluide intermédiaire tel que décrit dans la demande WO2012/120043.
Avantageusement, la température de l'étape de chauffage est comprise de 50°C à 100°C, de préférence de 60°C à 90°C.
Avantageusement, l'émulsion (E1) est également chauffée à une température comprise de 35°C à 150°C, de préférence de 50°C à 100°C, en particulier de 60°C à 90°C, avant la réalisation de l'étape b)susmentionnée de mise en contact de l'émulsion (E1) et du fluide huileux FI.
Selon une variante, l'émulsion (E1) est dénuée d'agent parfumant.
Selon une autre variante, l'émulsion (E1), notamment les gouttes (G1), comprend en outre au moins un agent parfumant.
Le procédé selon ce deuxième mode de réalisation peut également comprendre, après l'étape b) de formation des gouttes (G2) (et de préférence avant l'étape d)), une étape e) d'addition d'au moins une émulsion (E1') huile-dans-eau comprenant des gouttes (G1') comprenant au moins un composé sensible à la chaleur, lesdites gouttes (G1') comprenant une phase grasse et une écorce formée d'au moins un polymère anionique (PA1') et d'au moins un polymère cationique (PC1'), la taille des gouttes (G1') étant inférieure à 500 $\mu$m, voire inférieure à 200 $\mu$m. Les polymères anioniques (PA1') et les polymères cationiques (PC1') répondent aux définitions données ci-dessus pour (PA1) et (PC1) respectivement.
Un tel composé sensible à la chaleur peut par exemple être un agent parfumant, un colorant, un actif cosmétique, et leurs mélanges, de préférence un agent parfumant.
Avantageusement, cette étape e) d'addition d'au moins une émulsion (E1') est réalisée à température ambiante, de préférence après un retour de la composition obtenue en étape b) à température ambiante.
Selon un mode de réalisation, cette étape e) d'addition d'au moins une émulsion (E1') est effectuée par mélange, par exemple, via un mélangeur statique.
Ce deuxième mode de réalisation est particulièrement avantageux en ce qu'il constitue une alternative simplifiée par rapport au procédé selon le premier mode de réalisation ci-dessus (i.e. procédé n°1). En effet, le procédé selon ce deuxième mode de réalisation permet de s'affranchir de l'étape d) de filtration décrite précédemment mais surtout conduit directement à l'obtention d'une composition selon l'invention dans laquelle les gouttes (G2) sont dispersées de manière homogène. De fait, ce procédé n°2 permet un meilleur contrôle de la composition.
En d'autres termes, le procédé selon ce deuxième mode de réalisation ne nécessite pas d'étape de filtration ni de mélange à l'image des étapes e) et c) du procédé selon le premier mode de réalisation, et prévient donc toute détérioration/éclatement éventuel(le) des gouttes (G2).
Pour les procédés de préparation selon les premier et deuxième modes de réalisation décrits ci-dessus, lesdits procédés peuvent en outre comprendre :

- une étape d'injection d'une solution d'augmentation de la viscosité de la phase aqueuse continue de l'émulsion (E1), voire de l'émulsion (E2).

Selon un mode de réalisation, la solution d'augmentation de la viscosité comprend une base, notamment un hydroxyde d'alcalin, tel que l'hydroxyde de sodium.
De préférence, la solution d'augmentation de la viscosité est aqueuse.
Dans le procédé selon le premier mode de réalisation ci-dessus, cette étape d'ajout d'une solution d'augmentation de la viscosité est réalisée simultanément et/ou postérieurement à l'étape c) de mélange des émulsions (E1) et

(E2), de préférence postérieurement à cette étape c).

Dans le procédé selon le deuxième mode de réalisation ci-dessus, cette étape d'ajout d'une solution d'augmentation de la viscosité est réalisée après l'étape b) de formation des gouttes gélifiées (G2) et, le cas échéant, après l'étape e) susmentionnée.

- une étape d'injection d'une solution comprenant au moins un agent de texture tel que décrit précédemment pour faire varier la fluidité et/ou la sensorialité de la phase aqueuse de l'émulsion (E1), voire de l'émulsion (E2), et donc de la composition selon l'invention.

De préférence, cette solution est aqueuse et l'/les agent(s) de texture est/sont hydrophile(s).

Dans le procédé selon le premier mode de réalisation ci-dessus, cette étape est réalisée simultanément et/ou postérieurement à l'étape c) de mélange des émulsions (E1) et (E2), de préférence postérieurement à cette étape c).

Dans le procédé selon le deuxième mode de réalisation ci-dessus, cette étape est réalisée après l'étape b) de formation des gouttes (G2) et, le cas échéant, après l'étape e) susmentionnée.

- une étape de conditionnement de la composition ainsi obtenue dans tout dispositif adapté de packaging.

Enfin, l'homme du métier, au vu de ce qui précède, saura ajuster/adapter les procédés de préparation décrits ci-dessus dans l'hypothèse où la composition selon l'invention comprend en outre des gouttes (G3), voire des gouttes (G4), (G5), etc., le cas échéant gélifiées, mais différentes des gouttes (G2) telles que décrites précédemment.

***Utilisations***

De manière préférée, une composition selon l'invention est directement utilisable, à l'issue des procédés de préparation précités, à titre de composition, notamment cosmétique.

Les compositions selon l'invention peuvent notamment être utilisées dans le domaine cosmétique.

Elles peuvent comprendre, outre les ingrédients susmentionnés, au moins un milieu physiologiquement acceptable.

Par "milieu physiologiquement acceptable", on entend désigner un milieu convenant particulièrement à l'application d'une composition de l'invention sur les matières kératiniques, notamment la peau, les lèvres, les ongles, les cils ou les sourcils, et de préférence la peau.

Le milieu physiologiquement acceptable est généralement adapté à la nature du support sur lequel doit être appliquée la composition, ainsi qu'à l'aspect sous lequel la composition doit être conditionnée.

Selon un mode de réalisation, les compositions cosmétiques sont utilisées pour le maquillage et/ou le soin de matières kératiniques, notamment de la peau.

Les compositions cosmétiques selon l'invention peuvent être des produits de soin, de protection solaire, de nettoyage (démaquillage), d'hygiène ou de maquillage de la peau.

Ces compositions sont topiques et donc destinées à être appliquées notamment sur la peau.

Ainsi, la présente invention concerne également l'utilisation cosmétique non thérapeutique d'une composition cosmétique susmentionnée, comme produit de maquillage, d'hygiène, de nettoyage et/ou de soin de matières kératiniques, notamment de la peau.

En particulier, la présente invention concerne l'utilisation non thérapeutique d'une composition cosmétique selon l'invention pour le soin d'une matière kératinique, en particulier la peau, et en particulier pour prévenir et/ou traiter les signes cutanés du vieillissement, chronologique et/ou photo-induit, et/ou hydrater ladite matière kératinique, en particulier la peau.

Par « signes cutanés du vieillissement », et en particulier « signes du vieillissement de la peau », on entend, au sens de l'invention, toutes modifications de l'aspect extérieur de la peau dues au vieillissement, qu'il soit chronobiologique et/ou photo-induit, comme par exemple les rides et ridules, la peau flétrie, la peau molle, la peau amincie, le manque d'élasticité et/ou de tonus de la peau, le manque de densité et/ou de fermeté de la peau, mais également toutes modifications internes de la peau qui ne se traduisent pas systématiquement par un aspect extérieur modifié, comme par exemple toutes dégradations internes de la peau consécutives à une exposition aux rayonnements ultra-violets.

Selon un mode de réalisation, les compositions de l'invention sont sous la forme d'un fond de teint, d'un démaquillant, d'un soin du visage et/ou du corps et/ou du cheveu, d'un soin anti-âge, d'un protecteur solaire, d'un soin peau grasse, d'un soin whitening, d'un soin hydratant, d'une BB cream, d'une crème teintée ou d'un fond de teint, d'un nettoyant visage et/ou corps, d'un gel douche ou d'un shampoing.

Une composition de soin selon l'invention peut être en particulier une composition solaire, une crème de soin, un sérum ou un déodorant.

Les compositions selon l'invention peuvent être sous diverses formes, notamment sous forme de crème, de

baume, de lotion, de sérum, de gel, de gel-crème ou encore de brume.

La présente invention concerne également un procédé non thérapeutique de traitement cosmétique d'une matière kératinique, comprenant une étape d'application sur la matière kératinique d'au moins une couche d'une composition cosmétique telle que définie ci-dessus.

En particulier, la présente invention concerne un procédé non thérapeutique de traitement cosmétique de la peau, comprenant une étape d'application sur la peau d'au moins une couche d'une composition cosmétique telle que définie ci-dessus.

Dans toute la description, y compris les revendications, l'expression « comprenant un » doit être comprise comme étant synonyme de « comprenant au moins un », sauf si le contraire est spécifié.

Les expressions « compris entre ... et ... », « compris de ... à ... » et « allant de ... à ... » doivent se comprendre bornes incluses, sauf si le contraire est spécifié.

Les quantités des ingrédients figurant dans les exemples sont exprimées en pourcentage en poids par rapport au poids total de la composition, sauf indication contraire.

Les exemples qui suivent illustrent la présente invention sans en limiter la portée.

## EXEMPLES

### Exemple 1 : Compatibilité tensioactifs / gouttes gélifiées (G2)

La présente étude a pour objectif d'évaluer la compatibilité de gouttes gélifiées (G2) selon l'invention au contact de deux tensioactifs couramment mis en œuvre dans les phases aqueuses de crèmes cosmétiques.

Les gouttes (G2) considérées dans cette étude sont colorées en bleu afin de les visualiser dans les solutions de tensioactifs et dans les crèmes. Ces gouttes gélifiées (G2) ont été obtenues au moyen d'un dispositif microfluidique à chaud (environ 80°C) tel que décrit dans FR1558850 par mise en œuvre des phases aqueuse et grasse décrites dans le tableau ci-après :

| Nom | Nom INCI | *% w/w PHASES* | % w/w |
|---|---|---|---|
| **PHASE AQUEUSE** | | | |
| Eau osmosée | Aqua | 87,59 | 78,84 |
| Microcare PE | Phenoxyethanol | 0,89 | 0,80 |
| Microcare PTG | Pentylenglycol | 2,22 | 2,00 |
| Tego Carbomer 340 FD | Carbomer | 0,22 | 0,20 |
| Glycerine codex (99%) | Glycerin | 9,00 | 8,10 |
| EDETA BD | Disodium EDTA | 0,036 | 0,03 |
| Sodium Hydroxide Pellets PRS codex | Sodium Hydroxyde | 0,033 | 0,03 |
| | | ***100,00*** | ***90,00*** |
| **PHASE HUILEUSE** | | | |
| DUB ININ | Isononyl Isononanoate | 84,50 | 8,45 |
| Rheopearl KL2 | Dextrin Palmitate | 15,00 | 1,50 |
| Phat Blue DC6204 | CI 61565, CI 60725 | 0,0039 | 0,00039 |
| KF 8004 | Amodimethicone | 0,50 | 0,05 |
| | | ***100,00*** | ***10,00*** |

Une fois l'émulsion fabriquée, les gouttes gélifiées (G2) ont été isolées de la phase aqueuse par filtration avant les tests décrits ci-après.

Les gouttes gélifiées (G2) obtenues ont donc la composition suivante :

| Gouttes (G2) | | |
|---|---|---|
| DUB ININ | Isononyl Isononanoate | 84,50 |

(suite)

| Gouttes (G2) | | |
|---|---|---|
| Rheopearl KL2 | Dextrin Palmitate | 15,00 |
| Phat Blue DC6204 | CI 61565, CI 60725 | 0,0039 |
| KF 8004 | Amodimethicone | 0,50 |
| Tego Carbomer 340 FD | carbomer | * |
| **Total** | | ***100,00*** |
| ** Le carbomère utilisé est présent dans les gouttes (G2) uniquement au niveau de la membrane et donc de manière non quantifiable.* | | |

***A- Test avec deux tensioactifs***

Le comportement des gouttes gélifiées (G2) est étudié en présence des deux tensioactifs, à savoir le Montanov 68 EC et le Simulsol 165.

A cet effet, on prépare les quatre compositions décrites dans les deux tableaux ci-dessous :

| Nom | Nom INCI | % w/w |
|---|---|---|
| Eau osmosée | Aqua | Qsp* |
| Microcare PE | Phenoxyethanol | 0,39 |
| Microcare PTG | Pentylenglycol | 0,97 |
| Tego Carbomer 340FD | Carbomer | 0,06 |
| EDETA BD | Disodium EDTA | 0,01 |
| Sodium Hydroxide Pellets PRS codex | Sodium Hydroxyde | 0,01 |
| TENSIOACTIF (Montanov 68 EC **OU** Simulsol 165) | (Cetearyl alcohol & Cetearyl glucoside) **OU** (PEG 100 Stearate & Glyceryl Stearate) | ***2 - 10*** |
| Gouttes (G2) | Isononyl Isononanoate, CI 61565, CI 60725, coacervat (amodimethicone + carbomère), Dextrin Palmitate | 5,00 |
| **Total** | | **100,00** |
| * qsp : quantité suffisante pour | | |

avec :

| N° composition | |
|---|---|
| **183** | 2% Montanov 68 EC |
| **184** | 7% Montanov 68 EC |
| **185** | 5% Simulsol 165 |
| **186** | 10% Simulsol 165 |

Les gouttes (G2) ont été incorporées dans ces compositions par mélange manuel à l'aide d'une spatule pendant 30 secondes. La stabilité des gouttes (G2) pour ces formules 183, 184, 185 et 186 est observée pendant 1 mois à température ambiante (TA) et 50°C. Le test à 50°C a pour objectif de mettre la composition considérée dans des conditions de vieillissement accéléré.

Résultats

| Composition | T°C | OBSERVATIONS VISUELLES | TEXTURE des gouttes (G2) |
|---|---|---|---|
| **183 (2% Montanov 68 EC)** | **TA** | G2 bleues, sphériques, en suspension dans la phase continue. | Les G2 s'étalent bien sur la peau. |
| | **50°C** | G2 bleues, sphériques, ***plus petites que TA***, en suspension dans la phase continue plus visqueuse. | ***G2 plus résistantes (= huile gélifiée plus visqueuse) ; s'étalent plus difficilement.*** |
| **184 (7% Montanov 68 EC)** | **TA** | G2 bleues, sphériques, en suspension dans la phase continue. | Les G2 s'étalent bien sur la peau. |
| | **50°C** | G2 bleues, sphériques, ***plus petites que TA***, en suspension dans la phase continue plus visqueuse. | ***G2 plus résistantes (= huile gélifiée plus visqueuse) ; s'étalent plus difficilement.*** |
| **185 (5% Simulsol 165)** | **TA** | G2 bleues, sphériques, ***qui crèment en surface de la phase continue.*** | Les G2 s'étalent bien sur la peau. |
| | **50°C** | ***G2 bleues-vertes, sphériques, plus petites que TA et qui crèment plus vite qu'à TA.*** | ***G2 plus dures et certaines sont des grains solides difficiles à faire disparaître.*** |
| **186 (10% Simulsol 165)** | **TA** | G2 bleues, sphériques, en suspension dans la phase continue. | Les G2 s'étalent bien sur la peau. |
| | **50°C** | ***G2 vertes-bleues, sphériques, encore plus petites que 185 à 50°C et qui crèment en surface de la phase continue.*** | ***G2 plus dures et la plupart sont des grains solides difficiles à faire disparaître.*** |

De manière générale, on constate que la taille des gouttes (G2) diminue au cours du temps alors que leur viscosité augmente lorsque les compositions sont maintenues à 50°C.

***B- Test dans trois crèmes riches***

De la même manière que dans les compositions susmentionnées, les gouttes (G2) ci-dessus sont incorporées à 10% dans 3 crèmes riches différentes : CNBC-1 ; CNBC-2 ; CNBC-3.

*Composition de la crème riche CNBC-1*

| Nom | Nom INCI | % w/w |
|---|---|---|
| Eau osmosée | Aqua | qsp |
| Microcare PE | Phenoxyethanol | 0,8 |
| Microcare PTG | Pentylenglycol | 2,0 |
| Keltrol CGT | Xanthan Gum | 0,3 |
| Glycerin | Glycerin | 3,0 |
| **Montanov 68 EC** | Cetearyl alcohol & Cetearyl glucoside | 8,0 |
| Miglyol 812 N | Caprylic / Capric Triglycéride | 15,0 |
| Lipex Shea | Butyrospermum Parkii (Shea) Butter | 2,0 |
| JAUNE COVARINE W 1793 | CI 11710 (and) Glycerin (and) Aqua (and) Sodium Laureth Sulfate | 0,1 |
| **Total** | | **100,00** |

*Composition de la crème riche CNBC-2*

| Nom | Nom INCI | % w/w |
|---|---|---|
| Eau osmosée | Aqua | qsp |
| Microcare PE | Phenoxyethanol | 0,8 |
| Microcare PTG | Pentylenglycol | 2,0 |
| Satiaxane CX 911 | Xanthan Gum | 0,3 |
| Glycerin | Glycerin | 5,0 |
| **Simulsol 165** | PEG 100 Stearate & Glyceryl Stearate | 4,0 |
| Lipex 102 | Butyrospermum Parkii (Shea) Butter | 7,0 |
| Lanette 22 | Behenyl alcohol | 2,0 |
| Sweet Almond Oil | Prunus Amygdalus Dulcis Oil (Sweet Almond) | 12,0 |
| ROUGE COVARINE W 3792 | CI 73360 (and) Glycerin (and) Aqua (and) PVP | 0,1 |
| **Total** | | **100,00** |

*Composition de la crème riche CNBC-3*

| Nom | Nom INCI | % w/w |
|---|---|---|
| Eau osmosée | Aqua | qsp |
| Microcare PE | Phenoxyethanol | 0,8 |
| Microcare PTG | Pentylenglycol | 2,0 |
| Keltrol CGT | Xanthan Gum | 0,3 |
| Glycerin | Glycerin | 3,0 |
| **Montanov 68 EC** | Cetearyl alcohol & Cetearyl glucoside | 5,0 |
| Lanette 22 | Behenyl Alcohol | 13,0 |
| Lipex L'sens | Soybean Glycerides (and) Butyrospermum Parkii Butter Unsaponifiables | 2,0 |
| BLEU COVARINE W 6795 | CI 74160 (and) Glycerin (and) Aqua (and) Sodium Laureth Sulfate | 0,1 |
| **Total** | | **100,00** |

La stabilité des gouttes (G2) dans ces 3 compositions est également étudiée pendant 1 mois à température ambiante (TA) et 50°C.

Résultats

| Echantillon | T°C | OBSERVATIONS VISUELLES | TEXTURE |
|---|---|---|---|
| **CNBC-1** | **TA** | G2 bleues, sphériques ; Crème orangée | Représentative de la crème riche CNBC-1 |
| | **50°C** | G2 bleues, sphériques, ***mais avec une légère diminution de taille*** ; Crème orangée | ***Crème durcie, très consistante à la prise, quelques grains solides à l'application (=G2 plus dures)*** |
| **CNBC-2** | **TA** | G2 bleues, sphériques ; Crème rosée | Représentative de la crème riche CNBC-2 |
| | **50°C** | ***G2 beaucoup moins bleues que TA, sphériques, avec une diminution de taille*** ; Crème rosée | ***Toutes les G2 sont devenues des petits grains solides). La crème est plus fluide à l'application que TA.*** |

(suite)

| Echantillon | T°C | OBSERVATIONS VISUELLES | TEXTURE |
|---|---|---|---|
| **CNBC-3** | **TA** | G2 bleues, sphériques ; Crème bleutée | Représentative de la crème riche CNBC-3 |
| | **50°C** | G2 bleues, sphériques ; Crème bleutée ***qui tire sur le vert (=jaunissement)*** | ***Les G2 sont plus perceptibles qu'à TA ; isolées on sent que les gouttes (G2) sont plus consistantes qu'à TA.*** |

Là encore, on constate une tendance de gouttes (G2) à devenir plus petites et plus solides à 50°C.

**Conclusion**

La présente étude montre que la taille des gouttes (G2) diminue au cours du temps alors que leur viscosité/dureté augmente (augmentation de la concentration locale en gélifiant huileux) lorsque ces dernières sont en présence de tensioactifs ou de compositions en comprenant.

Ce phénomène se caractérise par un mûrissement compositionnel se traduisant par une migration d'une partie de la phase huileuse des gouttes (G2) vers les tensioactifs localisés dans la phase continue des crèmes. Ceci a pour effet de dégrader fortement les propriétés organoleptiques de la composition comprenant des gouttes (G2).

Eu égard aux contraintes de stabilité dans le temps requises dans le domaine cosmétique, ces résultats montrent l'impossibilité de mettre en œuvre de gouttes (G2) dans des compositions cosmétiques classiques (i.e. comprenant des tensioactifs).

**Exemple 2 : Préparation d'une composition selon l'invention avec le procédé n°1**

Une composition selon l'invention a été préparée en mélangeant d'une part, une émulsion (E1) comprenant des gouttes (G1) et, d'autre part, une émulsion (E2) comprenant des gouttes gélifiées (G2).

***1. Préparation de l'émulsion (E1)***

*a. Préparation d'un prémélange :*

- on a incorporé Lauroyl Lysine à la glycérine sous agitation puis agité le mélange pendant 10 minutes et vérifié l'absence d'agglomérats.

*b. Préparation d'une solution de soude 10% :*

- l'hydroxyde de sodium et l'eau osmosée ont été mélangées puis agitées pendant 10 minutes ; et
- on a vérifié l'homogénéité de la solution ainsi obtenue.

*c. Préparation de la phase aqueuse (OF1) :*

- on a incorporé l'eau osmosée puis le phénoxyéthanol, et ensuite le pentylène glycol, puis le disodium EDTA ;
- on a saupoudré en surface le carbomère TEGO CARBOMER 340 FD et CARBOPOL ULTREZ 21 POLYMER ;
- on a laissé reposer jusqu'à l'hydratation totale des carbomères (20-30 minutes) puis mis l'ensemble sous agitation à l'aide d'une défloculeuse ;
- on a agité jusqu'à la solubilisation totale des carbomères (environ 30 minutes) ;
- le prémélange susmentionné (a.) a été incorporé puis l'ensemble a été agité pendant 10 minutes ;
- la solution de soude susmentionnée (b.) a été ajoutée puis l'ensemble a été agité pendant 10 minutes ; et
- l'homogénéité de la solution a été vérifiée.

La phase aqueuse (OF1) ainsi obtenue comprend les constituants suivants :

| Nom | Nom INCI | % w/w |
|---|---|---|
| EAU OSMOSEE | Aqua | qsp |
| MICROCARE PE | Phenoxyethanol | 1,2346 |
| MICROCARE EMOLLIENT PTG | Pentyleneglycol | 2,7435 |
| TEGO CARBOMER 340 FD | Carbomer | 0,2333 |
| CARBOPOL ULTREZ 21 POLYMER | Acrylates/C10-30 Alkyl Acrylate Crosspolymer | 0,50 |
| GLYCERINE CODEX | Glycerin | 12,3457 |
| EDETA BD | Disodium EDTA | 0,0412 |
| AMIHOPE® LL | Lauroyl Lysine | 3,7037 |
| SOLUTION SOUDE à 10% | Sodium Hydroxyde | 0,1 |
| **Total** | | **100,00** |

*d. Préparation de la phase huileuse (IF1) :*

- on a incorporé l'amodiméthicone et l'isononanoate d'isononyle, puis on a agité doucement l'ensemble sans incorporer de bulles d'air ;
- on a vérifié l'homogénéité de la solution ;
- on a incorporé l'octyldodecanol puis on a agité pendant 3 minutes ;
- on a incorporé PLANTEC REFINED SHEA BUTTER ;
- le mélange ainsi obtenu a été chauffé sous agitation à 60°C pendant 5 minutes, puis on a vérifié l'homogénéité de la solution ;
- on a incorporé un parfum puis on a agité pendant 3 minutes;
- on a vérifié l'homogénéité de la solution.

La phase huileuse (IF1) ainsi obtenue comprend les constituants suivants :

| Nom | Nom INCI | % w/w |
|---|---|---|
| ISONONANOATE D'ISONONYLE - DUB ININ | Isononyl Isononanoate | qsp |
| EUTANOL G | Octyldodecanol | 19 |
| PLANTEC REFINED SHEA BUTTER | Butyrospermum Parkii | 19 |
| Parfum | Perfume | 3,33 |
| CAS-3131 | Amodimethicone | 0,2 |
| **Total** | | **100,00** |

*e. Préparation de la solution de base (BF1) :*

- l'hydroxyde de sodium et l'eau osmosée ont été mélangées puis agitées pendant 10 minutes ; et

- on a vérifié l'homogénéité de la solution ainsi obtenue.

La solution basique (BF1) ainsi obtenue comprend les constituants suivants :

| Nom | Nom INCI | % w/w |
|---|---|---|
| EAU OSMOSEE | Aqua | 89,90 |
| SOLUTION SOUDE à 10% | Sodium Hydroxyde | 10,10 |
| **Total** | | **100,00** |

*f. Préparation de l'émulsion (E1) :*

Pour préparer cette émulsion, on a utilisé 10% en poids de phase huileuse (IF1), 81% de phase aqueuse (OF1) et 9% de solution basique (BF1) :

- on a pesé la phase aqueuse (OF1) telle que décrite ci-dessus ;
- on a pesé la phase huileuse (IF1) telle que décrite ci-dessus ;
- on a ensuite placé séparément la phase huileuse et la phase aqueuse dans un bain-marie à 60°C pendant environ 15 minutes, tout en vérifiant que la température des deux phases est la même ;
- la phase aqueuse a été mise sous agitation ;
- on a incorporé progressivement la phase huileuse ;
- on a augmenté progressivement la vitesse d'agitation de manière à toujours avoir un vortex ;
- on a ensuite agité pendant 15 minutes ;
- on a ensuite refroidi à température ambiante sous agitation ;
- on a ajouté l'éthanol sous agitation ;
- après 10 min d'agitation la solution basique (BF1) a été incorporée et la vitesse d'agitation a été augmentée progressivement de manière à toujours avoir un vortex ;
- enfin, on a agité pendant 15 minutes en refroidissant le mélange avec un bain d'eau froide.

L'émulsion finale (E1) ainsi obtenue comprend les ingrédients suivants :

| Nom | Nom INCI | % w/w phases | % w/w *final* |
|---|---|---|---|
| **PHASE GEL AQUEUX** | | | |
| Eau osmosée | Aqua | qsp | qsp |
| Microcare PE | Phenoxyethanol | 1,11 | 1,00 |
| Microcare PTG | Pentylenqlycol | 2,47 | 2,22 |
| Tego Carbomer 340 FD | Carbomer | 0,21 | 0,19 |
| Carbopol Ultrez 21 Polymer | Acrylates/C10-30 Alkyl Acrylate Crosspolymer | 0,45 | 0,405 |
| Glycerine codex (99%) | Glycerin | 11,11 | 10,00 |
| EDETA BD | Disodium EDTA | 0,04 | 0,033 |

(suite)

| Nom | Nom INCI | % w/w phases | % w/w *final* |
|---|---|---|---|
| **PHASE GEL AQUEUX** | | | |
| AMIHOPE® LL | Lauroyl Lysine | 3,33 | 3,00 |
| Alcool Ethylique Dénaturé BITREX/TBA | Ethanol | 5,56 | 5,00 |
| Sodium Hydroxide Pellets PRS codex | Sodium Hydroxyde | 0,11 | 0,008 |
| **PHASE HUILEUSE** | | | |
| DUB ININ | Isononyl Isononanoate | 58,47 | 5,85 |
| EUTANOL G | Octyldodecanol | 19,00 | 1,90 |
| PLANTEC REFINED SHEA BUTTER | Butyrospermum Parkii | 19,00 | 1,90 |
| Parfum | Perfume | 3,33 | 0,33 |
| CAS-3131 | Amodimethicone | 0,20 | 0,02 |

***2. Préparation de l'émulsion (E2)***

*a. Préparation d'une solution de soude 10% :*

- l'hydroxyde de sodium et l'eau osmosée ont été mélangées puis agitées pendant 10 minutes ; et
- on a vérifié l'homogénéité de la solution ainsi obtenue.

*b. Préparation de la phase aqueuse (OF2) :*

- on a incorporé l'eau osmosée, le phénoxyéthanol, le pentylène glycol, et le disodium EDTA;
- on a saupoudré en surface le carbomère TEGO CARBOMER 340 FD-;
- on a laissé reposer jusqu'à l'hydratation totale du carbomère (environ 20-30 minutes) puis on a mis l'ensemble sous agitation à l'aide d'une défloculeuse ;
- on a agité jusqu'à la solubilisation totale du carbomère (environ 30 minutes) puis la glycérine a été incorporée ;
- l'ensemble a ensuite été agité pendant 5 minutes ;
- la solution de soude susmentionnée (a.) a été ajoutée puis l'ensemble a été agité pendant 10 minutes ; et
- l'homogénéité de la solution a été vérifiée.

La phase aqueuse (OF2) ainsi obtenue comprend les constituants suivants :

| Nom | Nom INCI | *% w/w* |
|---|---|---|
| Eau osmosée | Aqua | qsp |
| Microcare PE | Phenoxyethanol | 0,89 |
| Microcare PTG | Pentylenqlycol | 2,22 |
| Tego Carbomer 340 FD | Carbomer | 0,22 |
| Glycerine codex (99%) | Glycerin | 9,00 |
| EDETA BD | Disodium EDTA | 0,036 |
| Sodium Hydroxide Pellets PRS codex | Sodium Hydroxyde | 0,033 |
| | | ***100,00*** |

*c. Préparation de la phase huileuse (IF2) :*

- on a incorporé l'amodiméthicone et l'isononanoate d'isononyle puis on a agité doucement l'ensemble sans incorporer de bulles d'air ;
- on a chauffé ce mélange à 80°C ;
- en maintenant la température, on a incorporé le palmitate de dextrine;
- on a agité pendant 30 minutes ;
- on a vérifié l'homogénéité de la solution.

La phase huileuse (IF2) ainsi obtenue comprend les constituants suivants :

| Nom | Nom INCI | *% w/w* |
|---|---|---|
| DUB ININ | Isononyl Isononanoate | qsp |
| Rheopearl KL2 | Dextrin Palmitate | 15,00 |
| CAS-3131 | Amodimethicone | 0,20 |
| | | ***100,00*** |

*d. Préparation de l'émulsion (E2) :*

L'émulsion (E2) est préparée selon un procédé microfluidique à chaud (environ 80°C), notamment tel que décrit dans la demande FR1558850.

Les débits considérés (en mUh) à ce titre sont les suivants :

| | |
|---|---|
| OF2 | 150 |
| IF2 | 20 |

L'émulsion finale (E2) ainsi obtenue, comprenant des gouttes gélifiées (G2) monodisperses et de diamètre d'environ 800 μm, comprend les ingrédients suivants :

| Nom | Nom INCI | *% w/w phases* | % w/w Final |
|---|---|---|---|
| **PHASE AQUEUSE** | | | |
| Eau osmosée | Aqua | 87,5973 | 78,84 |
| Microcare PE | Phenoxyethanol | 0,8889 | 0,80 |
| Microcare PTG | Pentylenglycol | 2,2222 | 2,00 |
| Tego Carbomer 340 FD | Carbomer | 0,2222 | 0,20 |
| Glycerine codex (99%) | Glycerin | 9,00 | 8,10 |
| EDETA BD | Disodium EDTA | 0,0360 | 0,03 |
| Sodium Hydroxide Pellets PRS codex | Sodium Hydroxyde | 0,0333 | 0,030 |
| | | ***100,00*** | ***90,00*** |
| **PHASE HUILEUSE** | | | |
| DUB ININ | Isononyl Isononanoate | 84,80 | 8,48 |
| Rheopearl KL2 | Dextrin Palmitate | 15,00 | 1,50 |

(suite)

| PHASE HUILEUSE | | | |
|---|---|---|---|
| CAS-3131 | Amodimethicone | 0,20 | 0,02 |
| | | ***100,00*** | ***10,00*** |

***3. Préparation de la composition selon l'invention***

La composition selon l'invention est ensuite obtenue en mélangeant les émulsions (E1) et (E2) susmentionnées comme décrit ci-après.

L'émulsion (E2) est filtrée à l'aide d'une passoire ou un tamis dont la taille des pores est inférieure au diamètre des gouttes (G2) de manière à éliminer toute ou partie de la phase aqueuse (OF2). Les gouttes (G2) dans la passoire sont ensuite prélevées pour être introduites dans l'émulsion (E1) sous faible agitation.

Selon le visuel et la texture souhaitée, le ratio E1/E2 en masse varie ; ainsi, les gouttes (G2) peuvent représenter entre 1% et 20%, voire entre 5% et 15%, en poids par rapport au poids total de la composition selon l'invention.

**Exemple 3 : Préparation d'une composition selon l'invention avec le procédé n°2**

Un procédé de préparation d'une composition selon l'invention décrit dans cet exemple 3 diffère de celui décrit en exemple 2 en ce que les gouttes gélifiées (G2) sont fabriquées directement à partir d'une émulsion (E1).

***1. Préparation de l'émulsion (E1)***

Il s'agit de l'émulsion (E1) décrite en exemple 2 ci-dessus mais qui diffère par :

- l'absence de solution de base (BF1), de manière à conserver le caractère très fluide de cette émulsion (E1). Ainsi, l'émulsion (E1) a une viscosité inférieure à 3000 cPs, telle que mesurée à 25°C selon le protocole de mesure s'y rapportant décrit précédemment.
- l'ajout de CREASPERSE IRON BLUE dans la phase grasse (IF1) en fin du protocole de préparation de cette dernière, avec une homogénéisation pendant 5 minutes.

L'émulsion finale (E1) ainsi obtenue comprend les ingrédients suivants :

| Nom | Nom INCI | % w/w Phases | *% w/w final* |
|---|---|---|---|
| **PHASE GEL AQUEUX** | | | |
| Eau osmosée | Aqua | qsp | qsp |
| Microcare PE | Phenoxyethanol | 1,11 | 1,00 |
| Microcare PTG | Pentylenglycol | 2,47 | 2,222 |
| Tego Carbomer 340 FD | Carbomer | 0,210 | 0,189 |
| Carbopol Ultrez 21 Polymer | Acrylates/C10-30 Alkyl Acrylate Crosspolymer | 0,45 | 0,405 |
| Glycerine codex (99%) | Glycerin | 11,11 | 10,00 |
| EDETA BD | Disodium EDTA | 0,04 | 0,033 |
| AMIHOPE® LL | Lauroyl Lysine | 3,33 | 3,00 |
| Alcool Ethylique Dénaturé BITREX/TBA | Ethanol | 5,56 | 5,00 |
| Sodium Hydroxide Pellets PRS codex | Sodium Hydroxyde | 0,1 | 0,008 |
| **PHASE HUILEUSE** | | | |
| DUB ININ | Isononyl Isononanoate | 58,47 | 5,847 |

(suite)

| PHASE HUILEUSE | | | |
|---|---|---|---|
| EUTANOL G | Octyldodecanol | 19,00 | 1,90 |
| PLANTEC REFINED SHEA BUTTER | Butyrospermum Parkii | 19,00 | 1,90 |
| CREASPERSE IRON BLUE | CI 77510 (and) Hydrogenated Polydecene (and) Hydroxystearic Acid | 0,004 | 0,0036 |
| Parfum | Perfume | 3,33 | 0,333 |
| CAS-3131 | Amodimethicone | 0,20 | 0,02 |

***2. Préparation de la composition selon l'invention***

La composition selon l'invention est préparée selon un procédé microfluidique à chaud (environ 80°C), notamment tel que décrit dans la demande FR1558850 en utilisant l'émulsion (E1) ci-dessus comme phase aqueuse (OF) et la phase huileuse (IF2) décrite ci-après pour la formation des gouttes (G2).

| PHASE HUILEUSE (IF2) | | |
|---|---|---|
| DUB ININ | Isononyl Isononanoate | qsp |
| Rheopearl KL2 | Dextrin Palmitate | 15,00 |
| CREASPERSE IRON BLUE | CI 77510 (and) Hydrogenated Polydecene (and) Hydroxystearic Acid | 0,01 |
| CAS-3131 | Amodimethicone | 0,20 |
| ***TOTAL*** | | ***100,00*** |

La composition selon l'invention est préparée selon un procédé microfluidique à chaud (environ 80°C), notamment tel que décrit dans la demande FR1558850.

Les débits considérés (en mUh) à ce titre sont les suivants :

| | |
|---|---|
| OF (=émulsion (E1) | 150 |
| IF2 | 20 |

La fabrication des gouttes (G2) est donc simultanée à la fabrication de la composition selon l'invention. A l'issue de la fabrication de cette composition, une solution de base (BF1), identique à celle décrite en exemple 2, peut être ajoutée, de préférence par microfluidique, de manière à rehausser la viscosité de la phase aqueuse dans ladite composition selon l'invention, et ainsi suspendre les gouttes (G2).

Les compositions selon l'invention présentées en exemples 2 et 3 sont particulièrement avantageuses sur le plan visuel et sensoriel. Sur le plan visuel, le consommateur est face à des compositions comprenant des gouttes (G2) visibles à l'œil nu. Sur le plan sensoriel, la texture de ces compositions est évolutive. Les premiers instants d'application sont très aqueux avec un effet cassant marqué. Puis, le ressenti évolue vers un voile huileux qui s'estompe pour laisser une peau légère et hydratée. En parallèle, on sent les gouttes gélifiées (G2) fondre sous l'effet de l'étalement ce qui procure un effet huileux exacerbé.

**Exemple 4 : Préparation d'une composition transparente selon l'invention avec le procédé n°2**

La composition de l'exemple 4 diffère de celle décrite en exemple 3 en ce que la phase continue aqueuse et les gouttes (G1) et (G2) sont transparentes et la phase huileuse des gouttes (G2) est dénuée d'agent gélifiant.

***1. Préparation de l'émulsion (E1)***

| Nom | Nom INCI | % w/w |
|---|---|---|
| **Phase aqueuse (OF1)** | | |
| Eau osmosée | Aqua | qsp |
| MICROCARE PE | PHENOXYETHANOL, AQUA | 0,73 |
| MICROCARE EMOLLIENT PTG | PENTYLENE GLYCOL, AQUA | 1,82 |
| CARBOPOL ETD 2050 POLYMER | CARBOMER | 0,13 |
| GLYCERINE CODEX | GLYCERIN, AQUA | 22,76 |
| ZEMEA PROPANEDIOL | PROPANEDIOL | 13,65 |
| CARBOPOL ULTREZ 30 | Carbomer | 0,13 |
| NIACINAMIDE PC | Niacinamide | 4,55 |
| EDETA BD | DISODIUM EDTA | 0,038 |
| **Phase huileuse (IF1)** | | |
| CSF-3100 | Dimethicone | 10,63 |
| Parfum | Fragrance | 0,35 |
| CAS-3131 | Amodimethicone | 0,02 |
| **TOTAL** | | 100 |

***2. Préparation de la composition selon l'invention***

La phase huileuse (IF2) pour la formation des gouttes (G2) est décrite ci-après.

| **PHASE HUILEUSE (IF2)** | | |
|---|---|---|
| DUB ININ | Isononyl Isononanoate | qsp |
| Rheopearl KL2 (optionnel) | Dextrin palmitate | 0 ou 15 |
| CREASPERSE IRON BLUE (*optionnel*) | CI 77510 (and) Hydrogenated Polydecene (and) Hydroxystearic Acid | 0 ou 0,01 |
| CAS-3131 | Amodimethicone | 0,20 |
| ***TOTAL*** | | ***100,00*** |

Optionnellement, la phase huileuse (IF2) peut en outre comprendre au moins un agent gélifiant et/ou un agent colorant de manière à jouer sur le visuel des gouttes (G2).

Lorsque l'IF2 est dénuée d'agent gélifiant, le procédé de préparation de la composition selon l'exemple 4 diffère de celui décrit en exemple 3 en ce qu'il est réalisé à température ambiante ; ce procédé peut donc requérir avantageusement la mise en œuvre d'un fluide intermédiaire (FI) comprenant uniquement de l'Isononyl Isononanoate, comme décrit dans WO2012/120043.

Les débits considérés (en mUh) sont les suivants :

| | |
|---|---|
| OF (=émulsion (E1) | 150 |
| FI (optionnel) | 2 |
| IF2 | 20 |

La fabrication des gouttes (G2) est donc simultanée à la fabrication de la composition selon l'invention. A l'issue de la fabrication de cette composition, une solution de base (BF1), identique à celle décrite en exemple 2, peut être ajoutée, de préférence par microfluidique, de manière à rehausser la viscosité de la phase aqueuse dans ladite composition selon l'invention, et ainsi suspendre les gouttes (G2).

**Exemple 5** : **exemple d'une composition cosmétique**

Une composition telle que décrite dans le tableau ci-après peut être préparée selon l'un des procédés de fabrication décrit dans la présente invention et notamment selon le procédé décrit en exemple 3 ci-dessus.

| Nom | Nom INCI | *% w/w final* |
|---|---|---|
| **PHASE GEL AQUEUX** | | |
| Eau osmosée | Aqua | qsp |
| Microcare PE | Phenoxyethanol | 0.1-1 % |
| Microcare PTG | Pentylenglycol | 0.5-4% |
| Tego Carbomer 340 FD | Carbomer | 0.01-1% |
| Carbopol Ultrez 21 Polymer | Acrylates/C10-30 Alkyl Acrylate Crosspolymer | 0.1-2% |
| Glycerine codex (99%) | Glycerin | 1-15% |
| EDETA BD | Disodium EDTA | 0.01-0.5% |
| Alcool Ethylique Dénaturé BITREX/TBA | Ethanol | 1-6% |
| Sodium Hydroxide Pellets PRS codex | Sodium Hydroxyde | 0,001-0.1% |
| **PHASE HUILEUSE des gouttes (G1)** | | |
| DUB ININ | Isononyl Isononanoate | 1-10% |
| | Isostearyl Neopentanoate | 0.5-5% |
| | Isodecyl neopentanoate | 0.5-5% |
| | Isododecane | 0.5-5% |
| PLANTEC REFINED SHEA BUTTER | Butyrospermum Parkii | 0.5-5% |
| | Squalane | 0.1-5% |
| CREASPERSE IRON BLUE | CI 77510 (and) Hydrogenated Polydecene (and) Hydroxystearic Acid | 0-1% |
| Parfum | Perfume | 0,1-1 % |
| CAS-3131 | Amodimethicone | 0,01-0.5"% |
| **PHASE HUILEUSE des gouttes (G2)** | | |
| DUB ININ | Isononyl Isononanoate | 0.1-5% |
| | Isostearyl Neopentanoate | 0.5-5% |
| | Octyldodecyl myristate | 0.5-5% |
| | Neopentyl glycol diheptanoate and isododecane | 0.5-5% |
| Rheopearl KL2 | Dextrin Palmitate | 0.5-5% |
| CREASPERSE IRON BLUE | CI 77510 (and) Hydrogenated Polydecene (and) Hydroxystearic Acid | 0-1% |
| CAS-3131 | Amodimethicone | 0,01-0.5% |
| ***TOTAL*** | | ***100,00*** |

Une composition selon l'exemple 5 peut être appliquée chaque matin sur le visage pour hydrater, renforcer la

fonction barrière, protéger et apporter de l'éclat à la peau.

Il a été observé qu'une composition selon l'exemple 5, à l'usage, notamment comme indiqué ci-dessus, permet une micro-diffusion prolongée des actifs, en particulier des agents hydratants et anti-âge, et de fait procure une action continue au cœur de la peau.

## Revendications

1. Composition, notamment cosmétique, sous la forme d'une émulsion huile-dans-eau, comprenant une phase aqueuse continue et une phase grasse dispersée sous forme de gouttes (G1) et (G2),
lesdites gouttes (G1) comprenant une phase grasse et une écorce formée d'au moins un polymère anionique (PA1) et d'au moins un polymère cationique (PC1), la taille des gouttes (G1) étant inférieure à 500 μm, et
lesdites gouttes (G2) comprenant une phase grasse et une écorce, ladite écorce étant formée d'au moins un polymère anionique (PA2), identique ou différent de (PA1), et d'au moins un polymère cationique (PC2), identique ou différent de (PC1), la taille des gouttes (G2) étant supérieure à 500 μm.

2. Composition selon la revendication 1, dans laquelle la phase grasse des gouttes (G2) comprend au moins un agent gélifiant.

3. Composition selon l'une quelconque des revendications 1 ou 2, dans laquelle la phase grasse des gouttes (G1) et/ou des gouttes (G2) comprend au moins une huile (H1) choisie dans le groupe constitué des huiles hydrocarbonées d'origine animale, des esters et éthers de synthèse, des hydrocarbures linéaires ou ramifiés, d'origine minérale ou synthétique, des huiles de silicone, des alcools gras ayant de 8 à 26 atomes de carbone, des huiles fluorées partiellement hydrocarbonées et/ou siliconées et de leurs mélanges.

4. Composition selon l'une quelconque des revendications 1 à 3, dans laquelle les polymères anioniques (PA1) et (PA2), identiques ou différents, sont des polymères comprenant des unités monomériques comportant au moins une fonction acide carboxylique, de préférence sont choisis parmi les carbomères et les copolymères réticulés acrylates/$C_{10}$-$_{30}$ alkyl acrylate.

5. Composition selon l'une quelconque des revendications 1 à 4, dans laquelle la composition comprend de 0,01 % à 10%, de préférence de 0,05% à 5%, en poids de polymère(s) anionique(s) (PA1) et (PA2), notamment de carbomère(s), par rapport au poids total de ladite composition.

6. Composition selon l'une quelconque des revendications 1 à 5, dans laquelle les polymères cationiques (PC1) et (PC2), identiques ou différents, répondent à la formule suivante :
dans laquelle :

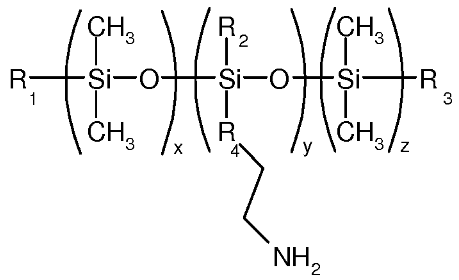

   - $R_1$, $R_2$ et $R_3$, indépendamment les uns des autres, représentent OH ou $CH_3$ ;
   - $R_4$ représente un groupe $-CH_2-$ ou un groupe -X-NH- dans lequel X est un radical alkylène divalent en C3 ou C4 ;
   - x est un nombre entier compris entre 10 et 5 000 ;
   - y est un nombre entier compris entre 2 et 1 000 ; et
   - z est un nombre entier compris entre 0 et 10.

7. Composition selon l'une quelconque des revendications 1 à 6, dans laquelle les gouttes (G1) et (G2) comprennent de 0,01% à 10%, de préférence de 0,05% à 5%, en poids de polymère(s) cationique(s) (PC1) et (PC2), notamment d'amodiméthicone(s), par rapport au poids total de la phase grasse des gouttes (G1) et (G2).

**8.** Composition selon l'une quelconque des revendications 2 à 7, dans laquelle l'agent gélifiant des gouttes (G2) est choisi dans le groupe constitué des agents gélifiants lipophiles organiques, minéraux, polymériques ou moléculaires ; des corps gras solides à température et pression ambiante ; et de leurs mélanges.

**9.** Composition selon la revendication 8, dans laquelle l'agent gélifiant est choisi dans le groupe constitué des polyacrylates, des esters de dextrine et d'acide(s) gras, des esters de glycérol et d'acide(s) gras, des polyamides, et de leurs mélanges.

**10.** Composition selon la revendication 9, dans laquelle les esters de dextrine et d'acide(s) gras sont choisis dans le groupe constitué des palmitates de dextrine, des myristates de dextrine, des palmitates/éthylhexanoates de dextrine, et de leurs mélanges.

**11.** Composition selon la revendication 8, dans laquelle l'agent gélifiant est choisi dans le groupe constitué des argiles modifiées, des silices, telles que la silice pyrogénée, et de leurs mélanges.

**12.** Composition selon l'une quelconque des revendications 1 à 11, dans laquelle ladite composition comprend un rapport pondéral « gouttes (G1) / gouttes (G2) » compris entre 0,03 et 50, de préférence entre 0,15 et 20, et mieux entre 0,33 et 10.

**13.** Composition selon l'une quelconque des revendications 1 à 12, comprenant en outre au moins un actif choisi parmi les agents hydratants, les agents cicatrisants, les agents dépigmentants, les filtres UV, les agents desquamants, les agents antioxydants, les actifs stimulant la synthèse des macromoléculaires dermiques et/ou épidermiques, les agents dermodécontractants, les agents anti-transpirants, les agents apaisants, les agents anti-âge, les agents parfumants et leurs mélanges.

**14.** Composition selon l'une quelconque des revendications 1 à 13, **caractérisée en ce qu'**elle ne comprend pas de tensioactif.

**15.** Composition selon l'une quelconque des revendications 1 à 14, **caractérisée en ce que** les gouttes (G1) et/ou (G2) comprennent en outre au moins un agent colorant.

**16.** Composition selon l'une quelconque des revendications 1 à 15, dans laquelle ladite composition comprend plus de 10%, de préférence plus de 15%, et mieux plus de 20%, en poids de phase grasse par rapport au poids total de ladite composition.

**17.** Procédé de préparation d'une composition selon l'une quelconque des revendications 1 à 16, comprenant les étapes suivantes :

a) la préparation d'une émulsion (E1) huile-dans-eau comprenant des gouttes (G1), par mélange sous agitation d'une phase grasse dans une phase aqueuse, ladite phase grasse comprenant au moins un polymère cationique (PC1) et ladite phase aqueuse comprenant au moins de l'eau et au moins un polymère anionique (PA1),
b) la préparation d'une émulsion (E2) huile-dans-eau comprenant des gouttes (G2), par mise en contact d'un fluide huileux FI, ledit fluide huileux FI comprenant au moins un polymère cationique (PC2), identique ou différent de (PC1), et optionnellement au moins un agent gélifiant, et d'un fluide aqueux FE, ledit fluide aqueux FE comprenant au moins de l'eau et au moins un polymère anionique (PA2), identique ou différent de (PA1), pour former des gouttes (G2) comprenant une phase grasse, constituée du fluide huileux FI, dispersées dans une phase aqueuse continue, constituée de fluide FE,
c) le mélange des émulsions (E1) et (E2), et
d) optionnellement, l'ajout d'une solution d'augmentation de la viscosité des phases aqueuses, de préférence comprenant une base, notamment un hydroxyde d'alcalin, tel que l'hydroxyde de sodium, et/ou d'une solution comprenant au moins un agent de texture des phases aqueuses.

**18.** Procédé de préparation d'une composition selon l'une quelconque des revendications 1 à 16, comprenant les étapes suivantes :

a) la préparation d'une émulsion (E1) huile-dans-eau comprenant des gouttes (G1), par mélange sous agitation d'une phase grasse dans une phase aqueuse, ladite phase grasse comprenant au moins un polymère cationique (PC1) et ladite phase aqueuse comprenant au moins de l'eau et au moins un polymère anionique (PA1),

b) la mise en contact de l'émulsion (E1) et d'un fluide huileux FI comprenant au moins un polymère cationique (PC2), identique ou différent de (PC1), et optionnellement au moins un agent gélifiant, et la formation des gouttes (G2) comprenant une phase grasse, constituée du fluide huileux FI, dispersées dans l'émulsion (E1) ; et
c) optionnellement, l'ajout d'une solution d'augmentation de la viscosité de la phase aqueuse, de préférence comprenant une base, notamment un hydroxyde d'alcalin, tel que l'hydroxyde de sodium, et/ou d'une solution comprenant au moins un agent de texture de la phase aqueuse.

**19.** Procédé non thérapeutique de traitement cosmétique d'une matière kératinique, en particulier de la peau, comprenant au moins une étape d'application sur ladite matière kératinique d'au moins une couche d'une composition selon l'une quelconque des revendications 1 à 12 et 14 à 16.

**20.** Utilisation non thérapeutique d'une composition cosmétique selon l'une quelconque des revendications 1 à 12 et 14 à 16 pour le soin d'une matière kératinique, en particulier la peau, et en particulier pour prévenir et/ou traiter les signes cutanés du vieillissement, chronologique et/ou photo-induit, et/ou hydrater ladite matière kératinique, en particulier la peau.

**21.** Utilisation d'une émulsion huile-dans-eau comprenant une phase aqueuse continue et une phase grasse dispersée sous forme de gouttes (G1) telles que définies dans l'une quelconque des revendications 1 à 7, 12 et 13, pour améliorer la stabilité et/ou préserver l'intégrité de gouttes (G2) telles que définies dans l'une quelconque des revendications 1 à 16.

## Patentansprüche

**1.** Zusammensetzung, insbesondere kosmetische Zusammensetzung, in Form einer Emulsion Öl-in-Wasser, die eine kontinuierliche wässrige Phase und eine Fettphase, die in Form von Tropfen (G1) und (G2) dispergiert ist, umfasst,

wobei die Tropfen (G1) eine Fettphase und eine aus mindestens einem anionischen Polymer (PA1) und mindestens einem kationischen Polymer (PC1) gebildete Schale enthalten, wobei Größe der Tropfen (G1) kleiner als 500 $\mu$m ist, und
die Tropfen (G2) eine Fettphase und eine aus mindestens einem anionischen Polymer (PA2), identisch oder unterschiedlich zu (PA1), und mindestens einem kationischen Polymer (PC2), identisch oder unterschiedlich zu (PA2), gebildeten Schale enthalten, wobei Größe der Tropfen (G2) größer als 500 $\mu$m ist.

**2.** Zusammensetzung nach Anspruch 1, bei der die Fettphase der Tropfen (G2) mindestens ein Geliermittel enthält.

**3.** Zusammensetzung nach einem beliebigen der Ansprüche 1 oder 2, bei der die Fettphase der Tropfen (G1) und/oder der Tropfen (G2) mindestens ein Öl (H1), ausgewählt aus der Gruppe, bestehend aus Kohlenwasserstoffölen tierischen Ursprungs, Estern und Syntheseethern, lineare oder verzweigte Kohlenwasserstoffe mineralischen oder synthetischen Ursprungs, Silikonölen, Fettalkoholen mit 8 bis 26 Kohlenstoffatomen, teilweise fluorierten Kohlenwasserstoffölen und/oder teilweise fluorierten Silikonölen und ihren Mischungen.

**4.** Zusammensetzung nach einem beliebigen der Ansprüche 1 bis 3, bei der die anionischen Polymere (PA1) und (PA2), identisch oder unterschiedlich, Polymere sind, die Monomereinheiten enthalten, die mindestens eine Carbonsäurefunktion aufweisen, vorzugsweise sind sie ausgewählt aus den Carbomeren und den vernetzten Acrylat-Copolymeren/$C_{10-30}$ Alkylacrylat-Copolymeren.

**5.** Zusammensetzung nach einem beliebigen der Ansprüche 1 bis 4, bei der die Zusammensetzung 0,01 Gew.% bis 10 Gew.%, vorzugsweise 0,05 Gew.% bis 5 Gew.% an anionischem(n) Polymer(en) (PA1) und (PA2), insbesondere an Carbomer(en), in Bezug auf das Gesamtgewicht der Zusammensetzung enthält.

**6.** Zusammensetzung nach einem beliebigen der Ansprüche 1 bis 5, bei der die kationischen Polymere (PC1) und (PC2), identisch oder unterschiedlich, der folgenden Formel entsprechen:

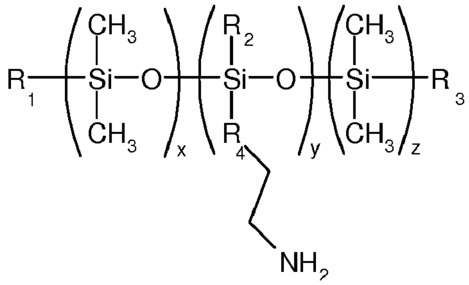

in der:

- $R_1$, $R_2$, und $R_3$, unabhängig voneinander OH oder $CH_3$ darstellen;
- $R_4$ eine Gruppe-$CH_2$- oder eine Gruppe -X-NH- darstellt, in der X ein divalenter C3 oder C4 Alkylenrest ist;
- x eine ganze Zahl zwischen 10 und 5.000 ist;
- y eine ganze Zahl zwischen 2 und 1.000 ist; und
- z eine ganze Zahl zwischen 0 und 10 ist.

**7.** Zusammensetzung nach einem beliebigen der Ansprüche 1 bis 4, bei der die Tropfen (G1) und (G2) 0,01 Gew.% bis 10 Gew.%, vorzugsweise 0,05 Gew.% bis 5 Gew.% an kationischem(n) Polymer(en) (PC1) und (PC2), insbesondere an Amodimethicon(en), in Bezug auf das Gesamtgewicht der Fettphase der Tropfen (G1) und (G2) enthalten.

**8.** Zusammensetzung nach einem beliebigen der Ansprüche 2 bis 7, bei der das Geliermittel der Tropfen (G2) ausgewählt ist aus der Gruppe, die besteht aus organischen, mineralischen, polymeren oder molekularen lipophilen Geliermitteln; bei Umgebungstemperatur und Umgebungsdruck festen Fettkörpern und ihren Mischungen.

**9.** Zusammensetzung nach Anspruch 8, bei der das Geliermittel ausgewählt ist aus der Gruppe, die besteht aus Polyacrylaten, Dextrin- und Fettsäureestern, Glycerin-und Fettsäureestern, Polyamiden und ihren Mischungen.

**10.** Zusammensetzung nach Anspruch 9, bei der die Dextrin- und Fettsäureester ausgewählt sind aus der Gruppe, die besteht aus Dextrin Palmitaten, Dextrin Myristaten, Dextrin Palmitaten/Ethylhexanoaten und ihren Mischungen.

**11.** Zusammensetzung nach Anspruch 8, bei dem das Geliermittel ausgewählt ist aus der Gruppe, die besteht aus modifizierten Tonen, Kieselsäuren, wie pyrogener Kieselsäure und ihren Mischungen.

**12.** Zusammensetzung nach einem beliebigen der Ansprüche 1 bis 11, bei der die Zusammensetzung ein Gewichtsverhältnis "Tropfen (G1) / Tropfen (G2)" zwischen 0,03 und 50, vorzugsweise zwischen 0,15 und 20 und besser zwischen 0,33 und 10 umfasst.

**13.** Zusammensetzung nach einem beliebigen der Ansprüche 1 bis 12, außerdem mindestens ein Aktivmittel, ausgewählt aus Feuchthaltemitteln, wundheilenden Mitteln, Depigmentierungmitteln, UV-Filtern, hautschälenden Mitteln, Antioxidantien, Aktivmitteln, die die Synthese der dermalen und/oder epidermalen Makromolekularen stimulieren, schweißhemmenden Mitteln, beruhigenden Mitteln, Anti-Aging Mitteln, Riechstoffen und ihren Mischungen.

**14.** Zusammensetzung nach einem der Ansprüche 1 bis 13, **dadurch gekennzeichnet, dass** sie kein Tensid enthält.

**15.** Zusammensetzung nach einem beliebigen der Ansprüche 1 bis 14, **dadurch gekennzeichnet, dass** Tropfen (G1) und/oder (G2) außerdem einen Farbmittel enthalten.

**16.** Zusammensetzung nach einem beliebigen der Ansprüche 1 bis 15, bei der die Zusammensetzung mehr als 10 Gew.%, vorzugsweise mehr als 15 Gew.% und besser mehr als 20 Gew.% in Bezug auf das Gesamtgewicht der Zusammensetzung enthält.

**17.** Verfahren zur Herstellung einer Zusammensetzung nach einem beliebigen der Ansprüche 1 bis 16, die folgenden Schritte umfassend:

a) Herstellen einer Tropfen (G1) enthaltenden Emulsion (E1) Öl-in-Wasser durch Mischung unter Rühren einer

Fettphase in einer wässrigen Phase, wobei die Fettphase mindestens ein kationisches Polymer (PC1) enthält und die wässrige Phase mindestens Wasser und mindestens ein anionisches Polymer (PA1) enthält,
b) Herstellen einer Tropfen (G1) enthaltenden Emulsion (E2) Öl-in-Wasser durch in Kontakt Bringen eines öligen Fluids, wobei das ölige Fluid mindestens ein kationisches Polymer (PC2), identisch oder unterschiedlich zu (PC1), enthält und optional mindestens ein Geliermittel, und eines wässrigen Fluids FE, wobei das wässriges Fluid FE mindestens Wasser und mindestens ein anionisches Polymer (PA2), identisch oder unterschiedlich zu (PA1), enthält, um Tropfen (G2) zu bilden, die eine aus dem öligen Fluid FI bestehende Fettphase enthalten und die in einer aus dem Fluid FE bestehenden kontinuierlichen wässrigen Phase dispergiert werden,
c) Mischen der Emulsionen (E1) und (E2) und
d) optional, Hinzufügen einer Lösung zum Erhöhen der Viskosität der wässrigen Phasen, die vorzugsweise eine Base enthält, insbesondere ein Alkalihydroxid, wie Natriumhydroxid, und/oder einer Lösung, die mindestens ein Mittel zur Texturierung der wässrigen Phasen enthält.

**18.** Verfahren zur Herstellung einer Zusammensetzung nach einem beliebigen der Ansprüche 1 bis 16, die folgenden Schritte umfassend:

a) Herstellen einer Tropfen (G1) enthaltenden Emulsion (E1) Öl-in-Wasser durch Mischung unter Rühren einer Fettphase in einer wässrigen Phase, wobei die Fettphase mindestens ein kationisches Polymer (PC1) enthält und die wässrige Phase mindestens Wasser und mindestens ein anionisches Polymer (PA1) enthält,
b) in Kontakt Bringen der Emulsion (E1) und eines öligen Fluids FI, das mindestens ein kationisches Polymer (PC2), identisch oder unterschiedlich zu (PC1), und optional mindestens ein Geliermittel enthält, und Bilden von in der Emulsion (E1) dispergierten Tropfen (G2), die eine Fettphase enthalten, die aus dem öligen Fluid FI besteht; und
c) optional, Hinzufügen einer Lösung zum Erhöhen der Viskosität der wässrigen Phase, vorzugsweise eine Base enthaltend, insbesondere ein Alkalihydroxid, wie Natriumhydroxid, und/oder einer Lösung, die mindestens ein Mittel zur Texturierung der wässrigen Phase enthält.

**19.** Nicht-therapeutisches Verfahren zur kosmetischen Behandlung eines Keratinmaterials, insbesondere der Haut, das mindestens einen Schritt des Aufbringens mindestens einer Schicht einer Zusammensetzung nach einem beliebigen der Ansprüche 1 bis 12 und 14 bis 16 auf das Keratinmaterial umfasst.

**20.** Nicht-therapeutische Verwendung einer kosmetischen Zusammensetzung nach einem beliebigen der Ansprüche 1 bis 12 und 14 bis 16 für die Pflege eines Keratinmaterials, insbesondere der Haut, und insbesondere zum Vermeiden und/oder Behandeln von Erscheinungen der chronologischen und/oder lichtbedingten Hautalterung, und/oder Zuführen von Feuchtigkeit dem Keratinmaterial, insbesondere der Haut.

**21.** Verwendung einer Emulsion Öl-in-Wasser, die eine kontinuierliche wässrige Phase und eine Fettphase enthält, die in Form von Tropfen (G1) dispergiert ist, wie sie in einem beliebigen der Ansprüche 1 bis 7, 12 und 13 definiert sind, um die Stabilität und/oder die Integrität der Tropfen (G2) zu verbessern bzw. zu bewahren, wie sie in einem beliebigen der Ansprüche 1 bis 16 definiert sind.

## Claims

**1.** Composition, in particular cosmetic, in the form of an oil-in-water emulsion, comprising a continuous aqueous phase and a dispersed oil phase in the form of drops (G1) and (G2),
wherein the drops (G1) comprise an oil phase and a shell formed of at least one anionic polymer (PA1) and at least one cationic polymer (PC1), wherein the size of the drops (G1) is less than 500 μm, and
wherein the drops (G2) comprise an oil phase and a shell, wherein the shell is formed of at least one anionic polymer (PA2), identical to or different from (PA1), and at least one cationic polymer (PC2), identical or different from (PC1), wherein the size of the drops (G2) is greater than 500 μm.

**2.** Composition according to claim 1, wherein the oil phase of the drops (G2) comprises at least one gelling agent.

**3.** Composition according to any one of the claims 1 or 2, wherein the oil phase of the drops (G1) and/or drops (G2) comprises at least one oil (H1) selected from the group consisting of hydrocarbon-based oils of animal origin, esters and synthetic ethers, linear or branched hydrocarbons of mineral or synthetic origin, silicone oils, fatty alcohols having from 8 to 26 carbon atoms, partially hydrocarbonated and/or siliconated fluorinated oils and their mixtures.

**4.** Composition according to any one of the claims 1 to 3, wherein the anionic polymers (PA1) and (PA2), which are identical or different, are polymers comprising monomeric units comprising at least one carboxylic acid function, and are preferably chosen from among crosslinked carbomers and acrylates/$C_{10-30}$ alkyl acrylate copolymers.

**5.** Composition according to any one of the claims 1 to 4, wherein the composition comprises from 0.01% to 10%, preferably from 0.05% to 5%, by weight of anionic polymer(s) (PA1) and (PA2), in particular carbomer(s), relative to the total weight of the composition.

**6.** Composition according to any one of the claims 1 to 5, wherein the cationic polymers (PC1) and (PC2), identical or different, correspond to the following formula:

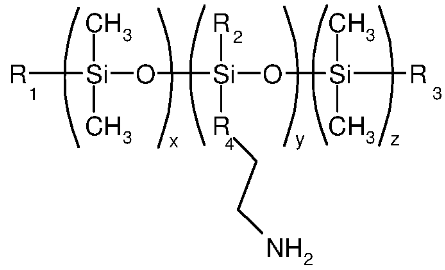

in which:

- $R_1$, $R_2$ and $R_3$, independently of each other, represent OH or $CH_3$;
- $R_4$ represents a -CH2- group or a -X-NH- group in which X is a C3 or C4 divalent alkylene radical;
- x is an integer from 10 to 5000;
- y is an integer between 2 and 1000; and
- z is an integer between 0 and 10.

**7.** Composition according to any one of the claims 1 to 6, wherein the drops (G1) and (G2) comprise from 0.01% to 10%, preferably from 0.05% to 5%, by weight of cationic polymer(s) (PC1) and (PC2), in particular amodimethicone(s), relative to the total weight of the oil phase of the drops (G1) and (G2).

**8.** Composition according to any one of the claims 2 to 7, wherein the gelling agent of the drops (G2) is selected from the group consisting of organic, inorganic, polymeric or molecular lipophilic gelling agents; fatty substances that are solid at room temperature and pressure; and their mixtures.

**9.** Composition according to claim 8 wherein the gelling agent is selected from the group consisting of polyacrylates, dextrin and fatty acid esters, esters of glycerol and fatty acid(s), polyamides, and mixtures thereof.

**10.** Composition according to claim 9, wherein the dextrin and fatty acid esters are selected from the group consisting of dextrin palmitates, dextrin myristates, dextrin palmitates/ethylhexanoates, and mixtures thereof.

**11.** Composition according to claim 8, wherein the gelling agent is selected from the group consisting of modified clays, silicas, such as fumed silica, and mixtures thereof.

**12.** Composition according to any one of the claims 1 to 11, wherein the composition comprises a weight ratio "drops (G1)/drops (G2)" of between 0.03 and 50, preferably between 0.15 and 20, and better still between 0.33 and 10.

**13.** Composition according to any one of the claims 1 to 12, further comprising at least one active agent selected from hydrating agents, healing agents, depigmenting agents, UV filters, desquamating agents, antioxidants, actives stimulating the synthesis of dermal and/or epidermal macromolecular agents, contracting agents, antiperspirants, soothing agents, anti-aging agents, perfuming agents and mixtures thereof.

**14.** Composition according to any one of the claims 1 to 13, **characterized in that** it does not include surfactant.

**15.** Composition according to any one of the claims 1 to 14, **characterized in that** the drops (G1) and/or (G2) further comprise at least one coloring agent.

**16.** Composition according to any one of the claims 1 to 15, wherein the composition comprises more than 10%, preferably more than 15%, and still more preferably more than 20%, by weight of oil phase relative to the total weight of the composition.

**17.** Method for the preparation of a composition according to any one of the claims 1 to 16, comprising the following steps:

a) the preparation of an oil-in-water emulsion (E1) comprising drops (G1), by stirring an oil phase in an aqueous phase, wherein the oil phase comprises at least one cationic polymer (PC1) and the aqueous phase comprises at least water and at least one anionic polymer (PA1),
b) the preparation of an oil-in-water emulsion (E2) comprising drops (G2), by contacting an oily fluid FI, wherein the oily fluid FI comprises at least one cationic polymer (PC2), identical or different from (PC1), and optionally at least one gelling agent, and an aqueous fluid FE, wherein the aqueous fluid FE comprises at least water and at least one anionic polymer (PA2), identical to or different from (PA1) to form drops (G2) comprising an oil phase, consisting of the oily fluid FI, dispersed in a continuous aqueous phase, consisting of FE fluid,
c) mixing the emulsions (E1) and (E2), and
d) optionally, adding a solution for increasing the viscosity of the aqueous phases, preferably comprising a base, in particular an alkaline hydroxide, such as sodium hydroxide, and/or a solution comprising at least one texturizing agent of the aqueous phases.

**18.** Method for the preparation of a composition according to any one of the claims 1 to 16, comprising the following steps:

a) the preparation of an oil-in-water emulsion (E1) comprising drops (G1), by stirring an oil phase in an aqueous phase, wherein the oil phase comprises at least one cationic polymer (PC1) and the aqueous phase comprises at least water and at least one anionic polymer (PA1),
b) contacting the emulsion (E1) with an oily fluid FI comprising at least one cationic polymer (PC2), identical to or different from (PC1), and optionally at least one gelling agent, and the formation of drops (G2) comprising an oil phase, consisting of the oily fluid FI, dispersed in the emulsion (E1); and
c) optionally, adding a solution for increasing the viscosity of the aqueous phase, preferably comprising a base, in particular an alkaline hydroxide, such as sodium hydroxide, and/or a solution comprising at least one texturizing agent of the aqueous phase.

**19.** Non-therapeutic method for the cosmetic treatment of a keratin material, in particular the skin, comprising at least one step of applying to the keratin material at least one layer of a composition according to any one of the claims 1 to 12 and 14 to 16.

**20.** Non-therapeutic use of a cosmetic composition according to any one of the claims 1 to 12 and 14 to 16 for the care of a keratin material, in particular the skin, and in particular for preventing and/or treating cutaneous signs of aging, chronological and/or photo-induced, and/or hydration of the keratin material, in particular the skin.

**21.** Use of an oil-in-water emulsion comprising a continuous aqueous phase and a dispersed oil phase in the form of drops (G1) as defined in any one of the claims 1 to 7, 12 and 13, to improve the stability and/or preserve the integrity of drops (G2) as defined in any one of the claims 1 to 16.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2012120043 A
- FR 2972367
- FR 2976824
- JP 2295912 A
- US 7470725 B
- WO 02056847 A
- WO 0247619 A
- US 5783657 A
- US 5874069 A
- US 5919441 A
- US 6051216 A
- US 5981680 A
- FR 2792190
- EP 2353577 A
- FR 1558848
- FR 1558849
- WO 2015055748 A
- FR 1558850


### Littérature non-brevet citée dans la description


- Silica silylate. CTFA, 2000
- Silica diméthyl silylate. CTFA, 2000
- Fats and Fatty Oils. **A. THOMAS.** Ullmann's Encyclopedia of Industrial Chemistry. 15 Juin 2000
- **SATO et al.** *J. Chem. Phys.,* 2007, vol. 111, 1393-1401
- handbook of Pharmaceutical Excipients. III